# EUROPEAN PATENT SPECIFICATION

(11) **EP 4 436 543 B1**
(45) Date of publication and mention of the grant of the patent: **03.09.2025**
(21) Application number: 22812827.8
(22) Date of filing: 21.11.2022
(51) Int. Cl.: A61K 9/00, A61K 9/70, A61K 47/32, A61K 47/20, A61K 47/12, A61K 38/095, A61K 38/26

(54) **MUCOSAL SUCTION PATCH AND USES THEREOF IN DRUG DELIVERY**
SCHLEIMHAUTSAUGPFLASTER UND VERWENDUNG DAVON ZUR ARZNEIMITTELABGABE
TIMBRE D'ASPIRATION MUQUEUSE ET SES UTILISATIONS DANS L'ADMINISTRATION DE MÉDICAMENTS

(30) Priority: 23.11.2021 EP 21209752
(43) Date of publication of application: 02.10.2024
(60) Divisional application: 25191634.2
(73) Proprietor: ETH Zurich, 8092 Zurich (CH)
(72) Inventor: LUO, Zhi, Shenzhen, Guangdong 518055 (CN); LEROUX, Jean-Christophe, 8053 Zurich (CH); KLEIN CERREJON, David, 8006 Zurich (CH)
(74) Representative: Regimbeau
(86) International application number: PCT/IB2022/061233
(87) International publication number: WO 2023/094971

(56) References cited:
- WO-A1-91/03271
- KR-A- 20210 012 215
- FRANCESCA TRAMACERE ET AL: "Artificial adhesion mechanisms inspired by octopus suckers", ROBOTICS AND AUTOMATION (ICRA), 2012 IEEE INTERNATIONAL CONFERENCE ON, IEEE, 14 May 2012 (2012-05-14), pages 3846 - 3851, XP032450789, ISBN: 978-1-4673-1403-9, DOI: 10.1109/ICRA.2012.6225058

## Description

### FIELD OF THE DISCLOSURE

The present disclosure relates to mucosal suction patches and uses thereof in non-parenteral drug delivery.

### BACKGROUND OF THE DISCLOSURE

Over the past few decades, advances in biotechnology and pharmaceutical sciences have led to the development and commercialization of a variety of new pharmaceuticals ranging from peptides, proteins, nucleic acids (e.g., RNAs) to novel chimeric molecules, such as proteolysis targeting chimeras (PROTACs). These compounds often exhibit superior properties compared to conventional low molecular weight drugs, such as target specificity and high potency. Their therapeutic indications also span a broad range of pathologies, for example, cardiovascular and immunological diseases, hepatitis, and cancer. However, the molecular weight (typically above 1000 Da) and the physicochemical nature (e.g., hydrophilicity, electrostatic charge) of these compounds often preclude their efficient absorption from the gastrointestinal (GI) tract and many of these compounds can degrade prematurely in the GI tract or during first pass metabolism. Consequently, these drugs are generally administered by parenteral routes, i.e., intra and extravascular injection, which often leads to low patient compliance as well as high treatment costs.

Great efforts have therefore been devoted to developing novel non-parenteral drug delivery systems for these macromolecular drugs (M. Sam, D. Brayden. "Formulation strategies to improve the efficacy of intestinal permeation enhancers." Adv. Drug Deliv. Rev (2021): 113925). However, success has been scarce. For example, in the case of peptides, only a few oral formulations for systemic delivery have entered clinical trials over the past 30 years, resulting in merely four of them reaching the market. Meanwhile, the oral bioavailability of most of these drugs remains very low (generally < 1%). As a result, novel and more efficient non-parenteral drug delivery strategies are of great interest to the pharmaceutical industry.

WO 91/03271 A1 discloses a mucosal patch comprising a dome enclosing a drug. The patch is attached with an adhesive.

There is a need for non-invasive (e.g., needle free) non-parenteral drug administration systems for enhancing systemic delivery, particularly of macromolecular drugs.

### SUMMARY OF THE DISCLOSURE

The present disclosure describes a drug delivery device that utilizes hypobaric pressure generated by a suction patch operation to achieve an efficient and modulable mucoadhesion and slight mucosa deformation and disruption. The patch enhances transmucosal drug delivery and absorption through the creation of a high concentration gradient at the mucosa patch adhesion site and the deformation of the mucosa to which it adheres.

The suction patch of the present disclosure has a tunable adhesion strength achieved through a pressure difference between the outside (ambient pressure) and the inside of the patch bulb(s) and eventually chemical interactions of the contact area of the patch with the mucosa. The adhesive strength is modulable by varying one or more of: (a) the bulb shape, including that defining (i) the encapsulated fluid volume (i.e., at least one cavity volume) (e.g., diameter of opening and depth of cavity); (ii) the encapsulated fluid volume shape (i.e., at least one cavity shape); (iii) the size of the patch area in contact with the mucosa (contact area(s)); (iv) the number of bulb(s); and (v) the at least one bulb(s) membrane thickness (e.g., which influences the bulb elasticity/flexibility and strength); (b) the patch (e.g., at least one bulb) material (e.g., which influences the patch elasticity/flexibility, strength, watertightness and airtightness); as well as (c) the eventual mucoadhesive coating and/or sealer on at least one bulb(s) of the suction patch.

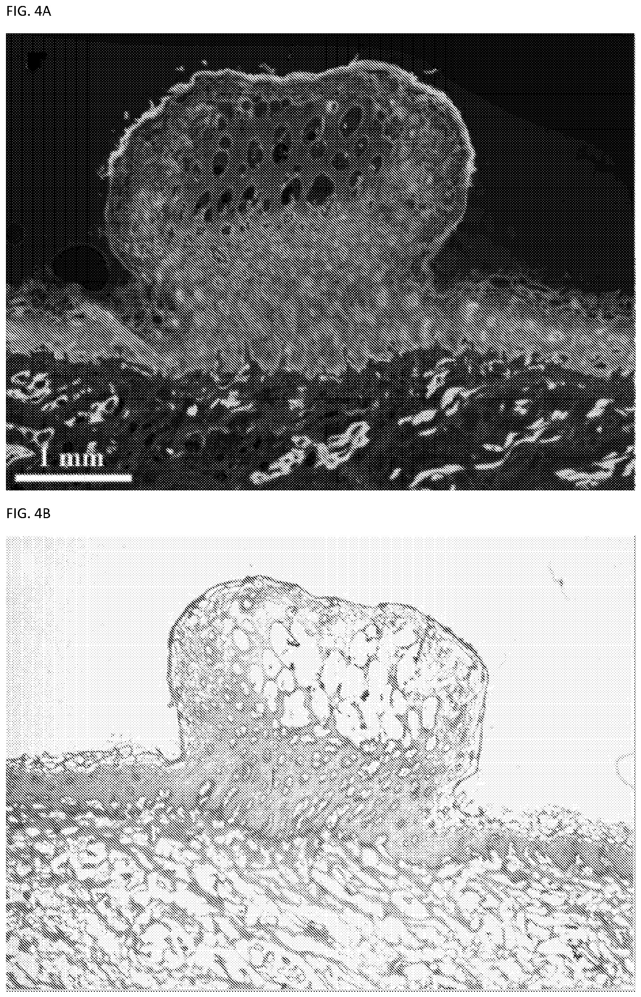

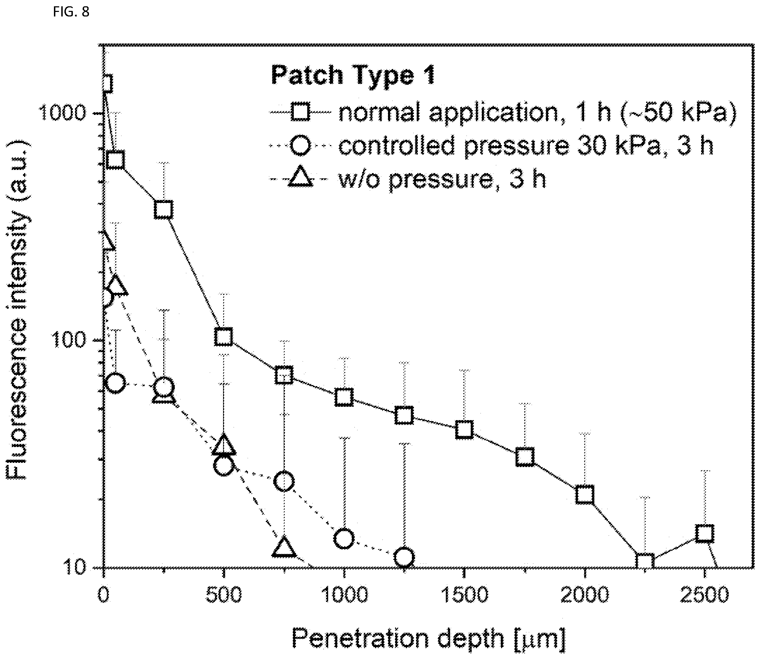

The suction strength generated by the hypobaric pressure and the resulting tissue deformation improves the permeation of the delivered drug by disrupting the mucosa, which improves the drug diffusion (See e.g., FIGs. 4 and 8) by, among others, enhancing the paracellular permeation.

### Mucosa Suction Patch

### Patch shape

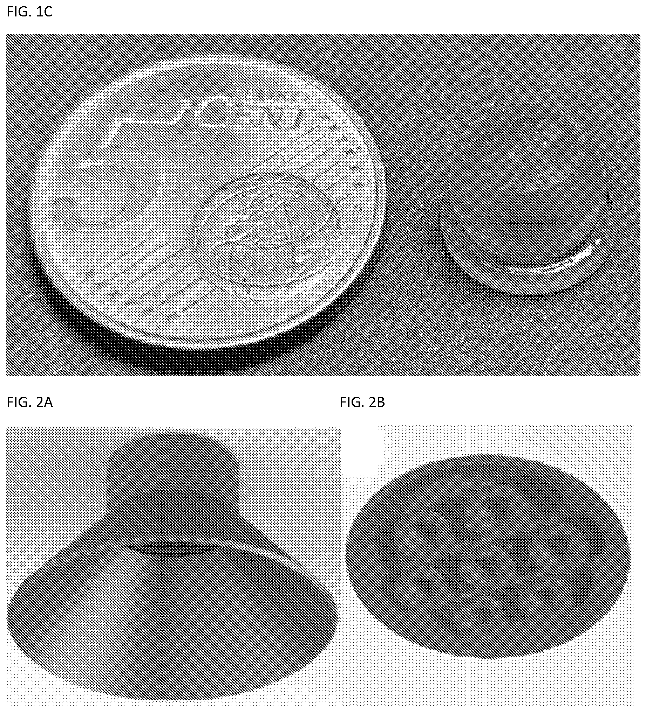

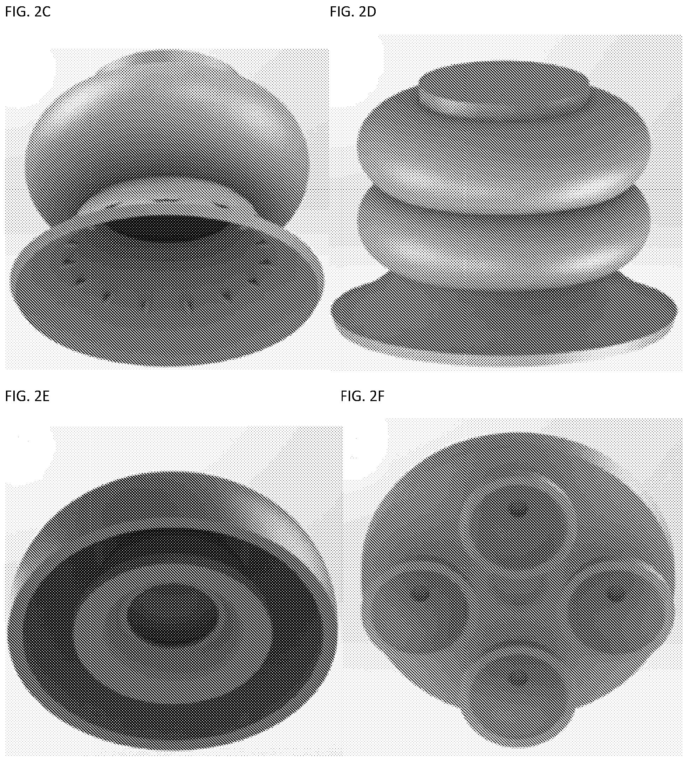

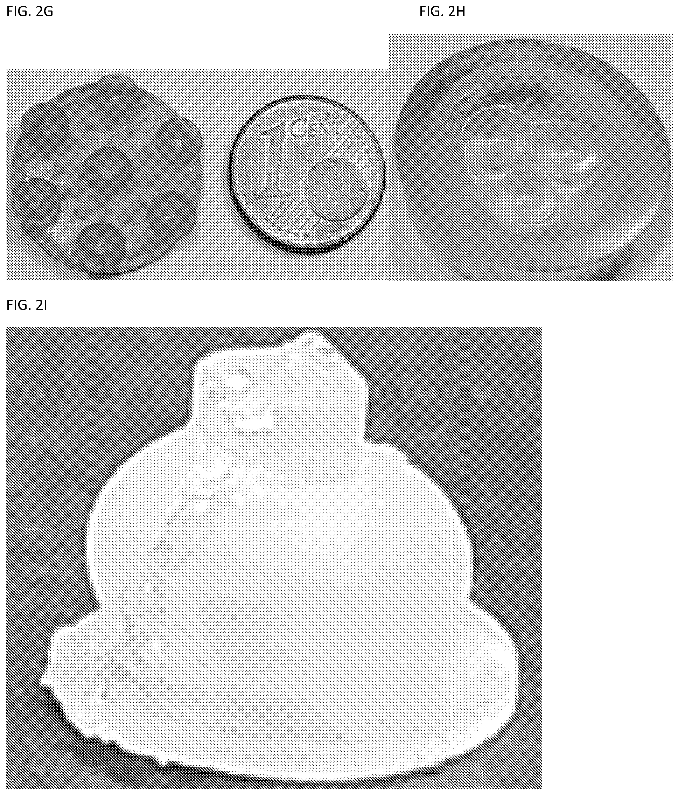

The patch comprises at least one cavity (i.e., one (a single) cavity or a plurality/array of at least 2, at least 3, at least 4, at least 5, at least 6, at least 7, at least 8, at least 9 or more cavities). The patch part(s) forming the cavity or plurality thereof is(are) designated herein the "bulb(s)" (i.e., suction component(s)). Each patch comprises at least one bulb (one or more bulb(s)). The patch has an external surface, including a (mechanical) pressure applying surface, an internal surface (comprised of the internal surfaces of one or more bulbs), and a mucosa sealing surface substantially opposite to the patch pressure applying surface. The patch mucosa sealing surface includes the rim(s) (contact area) of one or more of the bulb(s) that the patch comprises. The patch mucosa sealing surface delimits the opening(s) of the at least one bulb(s) and defines the patch base surface area. In patches containing a plurality of bulbs/cavities, the term "total base surface area" refers to the combined surface areas of all the bulbs openings. The shape of each of the one or more bulb(s) can be identical or different and be *e.g.,* cup shaped, cone shaped, etc. (See *e.g.,* FIGs. 2A-I). A larger bulb can contain one or more smaller bulbs in an array or core-shell configuration (*e.g.,* FIGs. 2B, 2E and 2H). Multiple bulbs can be connected by a backing layer, which may itself contain a compartment for drug(s) and/or excipient(s).

Each cavity has a volume and is in fluid communication (*e.g.,* air, drug formulation, sealing fluid, biological fluid) with at least one opening (*e.g.,* cup-shaped) to the outside. When the patch is in use on the mucosa, at least one opening is closed by the mucosa. In patches containing a plurality of bulbs/cavities, the term "total cavity volume" refers to the combined volume of all the patch's cavities. The total cavity volume is of a size sufficient to contain the drug formulation intended for delivery, and to, along with other features of the patch, enable the creation of a hypobaric (negative) pressure in the patch cavity(ies).

At least one, and up to all bulbs in the patch contain(s) a drug formulation. If the patch includes two or more bulbs that contain a drug formulation, the drug formulation may be identical or different in the two or more bulbs.

Each drug has specific delivery requirements (*e.g.,* small/larger surface area or larger/smaller mucosa deformation needed). The present disclosure encompasses the use of different bulb shapes to tailor different drug deliveries and/or adhesion strengths. For example, the present disclosure encompasses a patch comprising a larger bulb centrally (having a stronger adhesion to the mucosa) and smaller bulbs peripherally (having individually weaker adhesion to the mucosa).

In specific embodiments, the patch mucosa sealing surface may also include structural elements that (a) reduce the patch's undesired detachment from the mucosa (increase the patch's adhesion to the mucosa); (b) increase the shear and peeling forces, reduce lateral movement; and/or (c) a combination of (a) and (b).

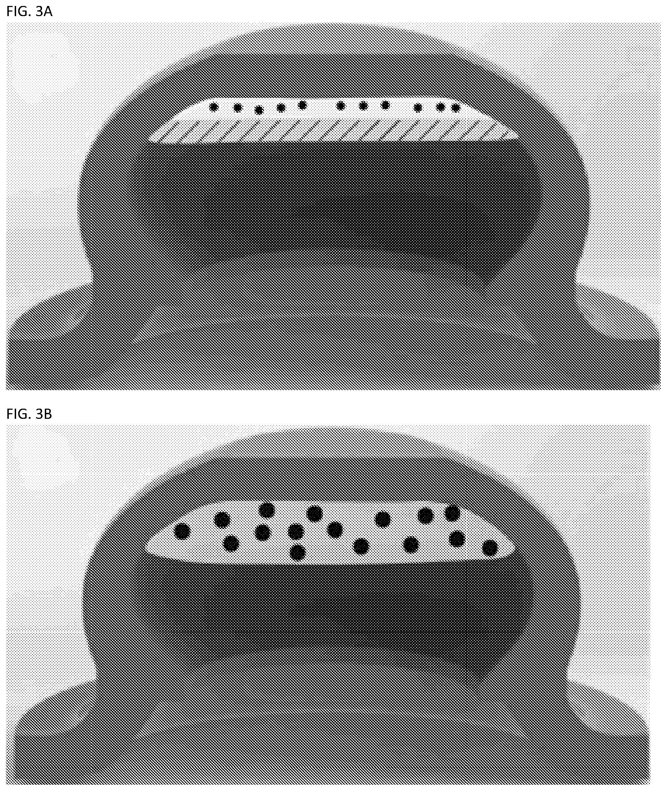

The cavities/internal surface of the patch comprises structural elements forming at least one drug loading compartment (e.g., FIGs. 3A-D). The drug loading compartment(s) is (are) designed so as to accommodate the desired amount of drug and carrier and/or excipient(s). The structural elements forming drug loading compartment(s) may be designed so as to not detrimentally reduce the adhesive strength of the patch.

The thickness of the patch bulb is designed to enable an easy finger mechanical pressing and releasing the pressure thereof. In a specific embodiment, the patch bulb material thickness of about 0.1 - 3 mm.

The patch pressure applying surface is the surface on which mechanical pressure (e.g., finger pressure) is applied when the patch mucosa sealing surface is placed on the mucosa to create suction. More particularly, as a result of the pressure, the one or more bulbs are distorted (collapse) and, when the mechanical pressure is released, the one or more bulbs substantially regain their original size and shape (expanded shape), temporarily creating negative pressure inside the one or more cavities, which in turn deforms (distorts) the mucosa which fills up at least part of the bulb(s).

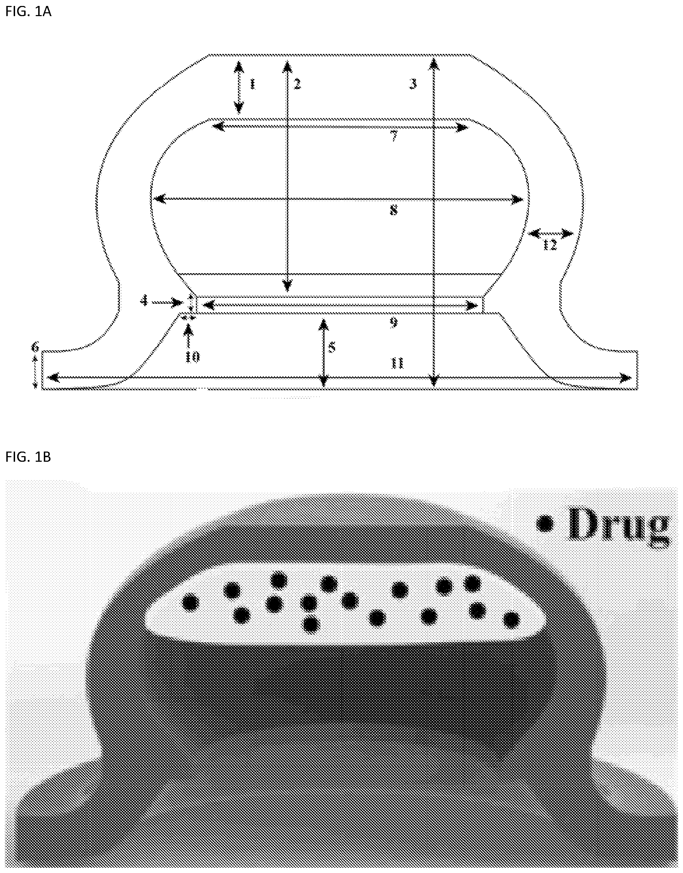

Without being so limited, various embodiments of patches of the present disclosure are shown in FIGs. 2A-I. Mucosal suction patches of the present disclosure are shown comprising a single bulb (See FIG. 1A-1C, 2A, 2C, 2D and 2I for example), a plurality/array of bulbs (see FIGs. 2F and 2G for example), and/or a core-shell structure with multiple bulbs (see FIGs. 2B, 2E and 2H for example). The patch can have a simple structure or a more complex structure.

When the patch comprises two or more adjacent bulbs, the patch may comprise a backing layer having a lower surface and an upper surface. The two or more bulbs are attached by their top surface (side opposite to their opening side) to the backing layer lower surface (e.g., FIG. 2F). The upper surface of the backing layer is, in such embodiment, the patch pressure applying surface. In patches devoid of a backing layer, the patch pressure applying surface can be the top surface of one of the at least one bulb (e.g., that of the single bulb or that of a larger bulb containing a least one smaller bulb).

In specific embodiments, the (i) total cavity volume of the at least one bulb and (ii) the total base surface area (all areas delimited by bulb rim(s)) are respectively of (i) 10 mm³ - 3000 mm³ (10 µL and 3000 µL) (total cavity volume) and (ii) 0.8-1200 mm² (total base surface area) where the patch comprises a plurality of bulbs (2 or more) or of 0.8 - 400 mm² when the patch comprises a single bulb.

The foregoing features are referred to herein as the patch shape.

The patch shape largely contributes to the patch's adhesion strength, negative pressure and the consequent degree of deformation of the mucosa, which impacts the permeation enhancing effects.

### Bulb elasticity

Elasticity is the ability of a body to resist a distorting influence and to return to its original size and shape when that influence or force is removed. The patch's one or more bulb(s) have a higher elastic modulus than that of the mucosa to which they are meant to adhere (*e.g.,* cheek mucosa). As a consequence, when the patch's mucosa sealing surface is placed on the mucosa and the mechanical pressure (distorting influence) applied on the at least one bulb is released, the at least one bulb regains at least part of its original size and shape (expanded state) and the mucosa is in turn deformed (distorted) and fills up part of the cavity volume of the at least one bulb. In specific embodiments, upon application of the patch on the mucosa, the total cavity volume of the patch is reduced at least by 10% (or at least 15% or at least 20%, or at least 25%, or at least 30% or at least 35%, or at least 40%, or at least 45%, or at least 50% or at least 55%, or at least 60%, or at least 65%, or at least 70% or at least 75%, or at least 80%, or at least 85%, or at least 90% or at least 95%).

In specific embodiments, the patch's at least one bulb has an elasticity of about 0.01 MPa to about 1000 MPa (about 0.1, 0.2, 0.5, 1, 2, 3, 4, 5, 6, 7, 10, 15, 20, 30, 40, 50, 75 MPa, to about 50, 100, 200, 300, 400, 500, 600, 700, 800 or 900 MPa) at a temperature of between about 20°C and about 55°C. In patches comprising more than one bulb, the bulbs may have substantially identical or different elasticities. In a specific embodiment, they have substantially identical elasticities.

### Patch size

The size of the patch is sufficiently small to be comfortably placed on the mucosa (*e.g.,* oral (*e.g.,* buccal) or vaginal mucosa) and sufficiently large to contain the desired drug formulation amount and encapsulate a sufficient amount of fluid to enable mucosa deformation and adhesion. Without being so limited, its external volume is between 50 µL and 5000 µL. The total cavity volume is between 10 µL and 3000 µL. In specific embodiments, the diameter of its total base surface area (including the mucosa sealing surface delimiting the opening(s)) is of about 1000 µm to about 40 mm when the patch comprises a plurality of bulbs and of about 1000 µm to about 22.5 mm when the patch comprises a single bulb). In specific embodiment, the patch's height (as measured from its base to its apex) is between about 1 and about 20 mm (or about 2 to about 20 mm, about 3 to about 20 mm, about 4 to about 20 mm, about 5 to about 20 mm, about 6 to about 20 mm, about 7 to about 20 mm, about 8 to about 20 mm, about 9 to about 20 mm, about 10 to about 20 mm; about 1 to about 19 mm, about 2 to about 19 mm, about 3 to about 19 mm, about 4 to about 19 mm, about 5 to about 19 mm, about 6 to about 19 mm, about 7 to about 19 mm, about 8 to about 19 mm, about 9 to about 19 mm, about 10 to about 19 mm; about 1 to about 18 mm, about 2 to about 18 mm, about 3 to about 18 mm, about 4 to about 18 mm, about 5 to about 18 mm, about 6 to about 18 mm, about 7 to about 18 mm, about 8 to about 18 mm, about 9 to about 18 mm, about 10 to about 18 mm; about 1 to about 15 mm, about 2 to about 15 mm, about 3 to about 15 mm, about 4 to about 15 mm, about 5 to about 15 mm, about 6 to about 15 mm, about 7 to about 15 mm, about 8 to about 15 mm, about 9 to about 15 mm, about 10 to about 15 mm; about 1 to about 10 mm, about 2 to about 10 mm, about 3 to about 10 mm, about 4 to about 10 mm, about 5 to about 10 mm, about 6 to about 10 mm, about 7 to about 10 mm, about 8 to about 10 mm, about 9 to about 10 mm, etc.).

The dimension of the patch can be easily adjusted according to the drug dose and patient needs. The dimension of each feature could be adjusted within a range. For example, if a single bulb/cavity patch as shown in FIG. 1A is used, potential size ranges for each part shown are as follows: Part 1: 0.1 - 3 mm; Part 2: 0.5 - 10 mm; Part 3: 1 - 25 mm; Part 4: 0.1 - 4 mm; Part 5: 0 - 7 mm; Part 6: 0.1 - 3 mm; Part 7: 1 - 30 mm; Part 8: 2 - 30 mm; Part 9: 1 - 30 mm; Part 10: 0 - 5 mm; Part 11: 2 - 30 mm; Part 12: 0.1 - 3 mm.

If a patch comprising a plurality of bulbs is used, potential size ranges for each part are as follows: number of bulbs per patch: 2 - 20; distance between centers of two contiguous individual bulbs: 3 - 20 mm; diameter of patch comprising plurality of bulbs : 6 - 40 mm.

### Patch material/Bulb material

The material of the at least one bulb of the patch is pharmaceutically acceptable. It is also sufficiently flexible/elastic to allow for its easy mechanical compression using fingers; and is watertight and airtight (i.e., sufficiently to create and maintain a negative pressure inside the patch and to retain the drug). The at least one bulb material comprises or consists of an elastomer. The bulb material is such as to confer to the one or more bulbs an elasticity of about 0.01 MPa to about 1000 MPa. Without being so limited, materials encompassed by the present disclosure include elastomers *e.g.,* polyester-based elastomers, such as poly(ε-caprolactone), poly(glycolide), poly(lactide), poly(hydroxyalkanoate), poly(4-hydroxybutyrate), poly(dioxanone), poly(1,3-trimethylene carbonate), poly(ethylene succinate), polybutylene terephthalate, and their copolymers; polyether-based elastomers, such as poly(ethyleneglycol), poly(tetramethylene) glycol, poly(propylene oxide), poly(p-phenylene oxide), polyaryletherketone, polyether ether ketone, polyphenyl ether, polytetrahydrofuran, and their copolymers; polyamides-based elastomers; polyurethanes-based elastomers; poly(ester amide)-based elastomers; polysaccharides-based elastomers; poly(β-amino ester)-based elastomers; poly(β-thioether ester)-based elastomers; polydimethylsiloxane (PDMS)-based elastomers; polyacrylates-based elastomers; synthetic and natural rubbers such as polybutadiene; copolymers (block, diblock, or triblock) of at least two thereof; or a mixture of at least two of these materials. In specific embodiments, the material is non biodegradable (*e.g.,* polyether-based elastomers; polydimethylsiloxane (PDMS)-based elastomers; polyacrylates-based elastomers; synthetic and natural rubbers such as polybutadiene). In other specific embodiments, the material is biodegradable (*e.g.,* polyester-based elastomers, polyamides-based elastomers; polyurethanes-based elastomers; poly(ester amide)-based elastomers; polysaccharides-based elastomers; poly(β-amino ester)-based elastomers; poly(β-thioether ester)-based elastomers). In a specific embodiment, the at least one bulb is an elastomer that is a polyester-based elastomer, polyether-based elastomer, polyamide-based elastomer, polyurethane-based elastomer; poly(ester amide)-based elastomer; polysaccharide-based elastomer; poly (β-amino ester)-based elastomer; poly(β-thioether ester)-based elastomer; polydimethylsiloxane (PDMS)-based elastomer; polyacrylate-based elastomer; synthetic rubber, natural rubber, a copolymer of at least two thereof; or a mixture of at least two of thereof.

The patch can be made of a single material (i.e., the material of the at least one bulb) or multiple materials, *e.g.,* a first material for the at least one bulb and a second material for the backing layer; different materials for different bulbs, different material in a single bulb, etc. The patch material(s) may be biodegradable or nonbiodegradable. The patch material is pharmaceutically acceptable.

The optional biodegradability of the patch in the gastrointestinal tract avoids risks associated with the patch's accidental swallowing.

In specific embodiments, the suction patch may be produced by 3D printing and molding.

### Patch coating/sealer

In specific embodiments, the patch mucosa sealing surface can be coated with a pharmaceutically acceptable mucoadhesive compound or composition (mucoadhesive coating) to further modulate (*e.g.,* increase) the patch adhesion strength. Without being so limited, such compounds and compositions include mucoadhesive polymers such as carbomers, polysaccharides such as chitosan and cellulose derivatives, poly(acrylic acid) based polymers, proteins/glycoproteins such as lectins, thiomers, polydopamine, poly(vinly alcohol) (PVA), alginate, poly(methacrylic acid) based polymers, various gums, poly(N-vinylpyrrolidone), and their co-polymers.

In other specific embodiment, a sealer can be added on the patch (immediately) prior its use so as to cover its mucosa sealing surface. The sealer is pharmaceutically acceptable or food grade. In specific embodiment, the sealer has a viscosity of about 0.98 mPa.s to about 1000 Pa.s. Without being so limited, in specific embodiments, the sealer is water. In other embodiments, the mucosal patch is placed on the mucosa without adding a sealer.

Such mucoadhesive coating and/or sealer can further increase the patch's adhesion to the mucosa by (a) improving the patches shear or peeling resistance; (b) reducing air/liquid leakage; and/or (c) a combination and (a) and (b).

### Adhesion strength

As used herein, the adhesion strength is meant to refer to the force needed to detach the patch from its substrate, *e.g.,* mucosa such as oral (*e.g.,* buccal) mucosa, normalized by the surface area of the inner rim (opening) of the at least one bulb or the total base surface area of the inner rims (openings) of more than one or all bulbs. The strength should be high enough to ensure the patch adhesion on the mucosa for a time sufficient for the drug to be released, and low enough to avoid bleeding or ulceration of the mucosa.

Patches of the present disclosure usefully possess an adhesion strength of about 0.5 kPa to about 200 kPa (i.e., about 0.05 N/cm² to 20 N/cm²). Such strength is derived at least in part from the negative pressure achieved in the patch (*e.g.,* up to ~100 kPa) and eventually in part from the patch mucoadhesive coating and/or sealer, which can increase the adhesion strength (*e.g.,* up to ~150 kPa). Without being so limited, various patch designs of the present disclosure that were tested *ex vivo* on porcine buccal tissue reproducibly achieved a suction strength of ~50 kPa (*e.g.,* patch with a design similar to that shown in FIG. 1, with 0.5 cm in diameter at the contact area). In specific embodiments, the adhesion strength is of about 5 kPa to about 150 kPA, about 10 kPa to about 150 kPa, about 15 kPa to about 150 kPa, about 20 kPa to about 150 kPA; about 5 kPa to about 120 kPa, about 10 kPa to about 120 kPa, about 15 kPa to about 120 kPa, about 20 kPa to about 120 kPa; about 5 kPa to about 110 kPa, about 10 kPa to about 110 kPa, about 15 kPa to about 110 kPa, about 20 kPa to about 110 kPa; about 5 kPa to about 100 kPa, about 10 kPa to about 100 kPa, about 15 kPa to about 100 kPa, or about 20 kPa to about 100 kPa.

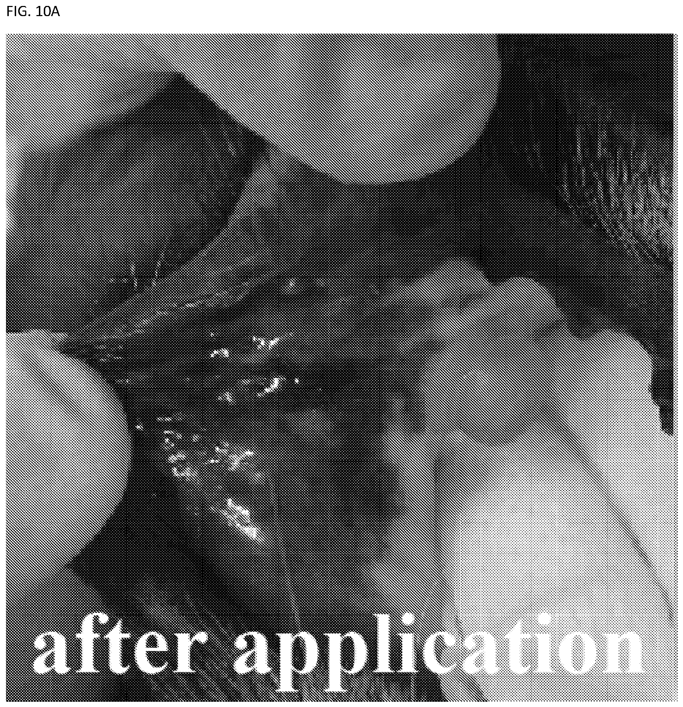

The patches of the present disclosure may remain firmly attached (adhere) to the oral (*e.g.,* buccal) mucosa for at least 1 min and up to 10 hours. In specific embodiments, they remain attached to the oral (*e.g.,* buccal) mucosa for 1-5 min; 1-10 min; 1-15 min; 1-30 min; 5-10 min; 5-15 min; 5-30 min; 10-30 min; 10-60 min; 15-30 min; 15-45 min; 15-60 min; 15-180 min; 30-60 min; 30-180 min; 1-180 min; 2-5 h; 2-10 h; and 5-10 h. They can be easily removed without causing tissue damage by controlled air leaking (See *e.g.,* FIGs. 10A-C).

### Drug

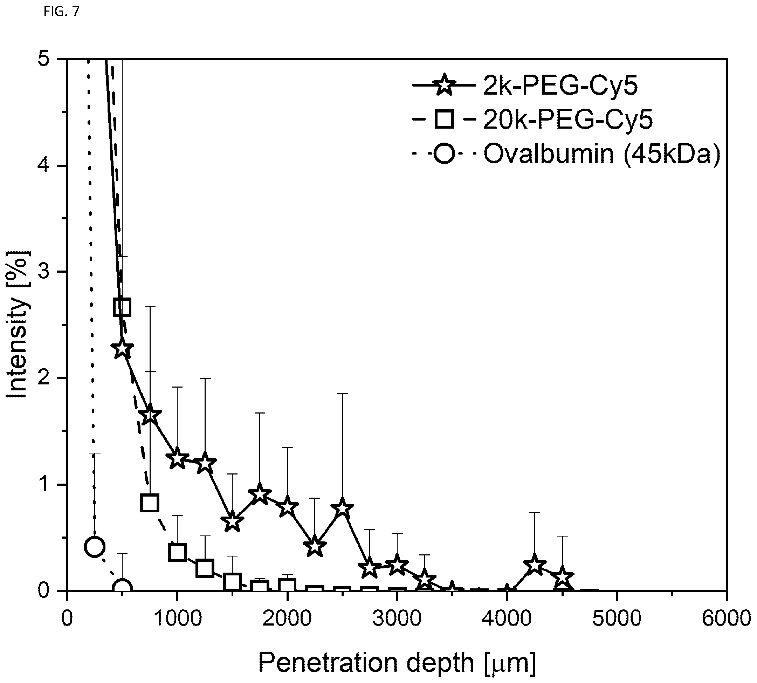

The term "drug(s)" as used herein refers to any pharmacologically active molecule or mixture of molecules (including non approved active pharmaceutical ingredients (APIs)). As used herein, mixture of molecules display pharmacological activity. Without being so limited, biological extracts such as plant or animal extracts are encompassed by the term mixture of molecules, and thereby by the term drug. Drugs that can be incorporated into the patch of the present disclosure can be low molecular weight active pharmaceutical ingredients (< 1000 Da) or large molecular weight active pharmaceutical ingredients (>1000 Da) such as polysaccharides, peptides, proteins, nucleic acids, dendrimers, polymer-drug conjugates, proteolysis-targeting chimeras (PROTACs); Lysosome-targeting chimaras (LYTAC); antibody-based PROTACs (AbTAC), antibody-drug conjugates, lipid or polymeric nanoparticles, exosomes, and vaccine antigens (see *e.g.,* FIGs. 7 and 11-14). While patches are most advantageously used to deliver drugs with high molecular weight (without being so limited, *e.g.,* between 1kDA and 50 kDa; *e.g.,* about 1 to about 45 kDa) or size (without being so limited between 0.5 nm and 10 nm; or between about 0.5 nm and 6 nm) as such drugs typically display low oral absorption and require injection, there are no lower size limitations to drugs that can be used in the patches of the present disclosure. The patches of the disclosure would also advantageously be used for drugs displaying low oral bioavailability related to degradation in the GI tract and/or poor diffusibility (poor absorption) across the mucosa, including drugs smaller than 1 kDa, and including cannabis oils containing *e.g.,* cannabidiol and/or cannabigerol and/or tetrahydrocannabinol.

Drugs that would particularly benefit from the delivery device of the present disclosure include the following: (1) semaglutide, an anti-diabetic and anti-obesity medication that acts like human glucagon-like peptide-1. The drug has a molecular weight of 4113 Da with an oral bioavailability ~1% even in the presence of large amount of permeation enhancers as excipients. The recently approved oral tablet ((Rybelsus) from Novo Nordisk for diabetes) also requires the drug to be taken at a specific time before breakfast every day, as the absorption is affected by food in the stomach. (2) octreotride has an oral bioavailability of 0.5-1% even in the presence of permeation enhancers; (3) human growth hormone, a drug to treat children's growth disorders and adult growth hormone deficiency. As is typical of protein drugs with a high molecular weight (22 kDa) which is prone to degradatioin in the GI tract, there are currently no oral formulations of this drug on the market; (4) insulin is currently only available for injection or pulmonary administration; (5) TNF-alpha inhibitors are antibodies that need to be injected. Other peptide and protein (macromolecular drugs) drugs with low oral bioavailability and high sensitivity to degradation in the GI-tract: (6) PROTACs, a new type of drug molecule that can degrade the target pathogenic proteins and regulate the related signaling pathways. With similar molecular weight to peptide-based drugs, the PROTACs often have low oral bioavailability. The patch could avoid several modifications to achieve stability and absorption via the GI tract; and (7) vaccines: As injected vaccines only trigger systemic immunization; mucosal immunity could also or alternatively be stimulated via the transmucosal route.

In view of the size limitation of the patch, and consequently limited drug compartment(s) size, the patch may be used to deliver drugs that can usefully be administered at a dose lower than about 100 mg (or lower than about 50 mg).

### Drug formulation

The drug(s) can be loaded in the patch with at least one excipient (one or more excipient(s)) and/or at least one carrier/solvent (one or more carrier(s) or solvent(s)) to form one or more drug formulations, with or without additional separately loaded drug free formulations comprising at least one excipient (one or more excipient(s)) such as carriers and solvents (one or more carrier(s) or solvent(s)) (in cavities/compartments separate from that or those where the drug or drugs are loaded). Each of those drug formulations and excipients/carriers/solvents formulations can independently be in a solid, organogel, hydrogel, suspension, paste, or liquid/solution state. The one or more formulations can be released simultaneously or sequentially. The one or more formulations can be loaded as mixtures, in a layered structure, or in separated compartments (FIGs. 3A-D).

Various dispersion systems (carriers and/or excipients) could be used to load the drugs inside the patch, such as but not limited to PVA, glycerol, hydrogel, poly(ethylene glycol), fatty acids, (poly)saccharides (*e.g.,* cyclodextrins), and waxes. Without being so limited, such carriers and/or excipients may be used to create bulk volume and assist in controlling the release rate.

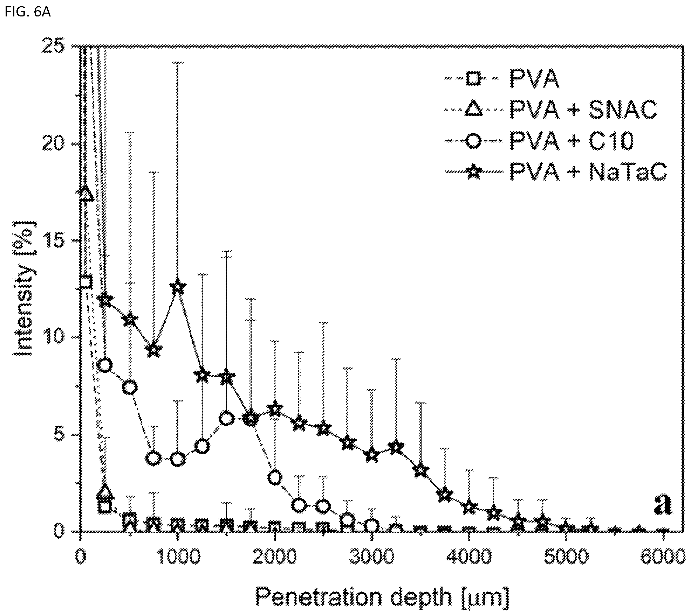

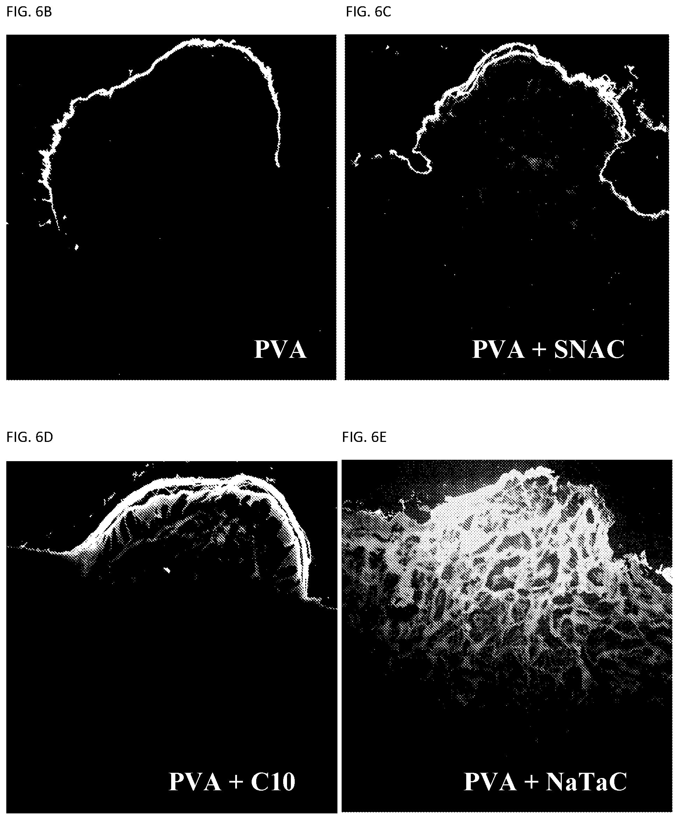

Without being so limited, excipients such as permeation enhancers can be loaded inside one or more compartment(s) (cavities/bulbs) of the patch. The combination of chemical disruption produced by a permeation enhancer with the mechanical stretching (mucosa deformation) caused by the mechanical suction can further promote the diffusion of the drug (*e.g.,* hydrophilic macromolecules) (see *e.g.,* FIGs. 6-8, 11-14).

As used herein, the permeation enhancers that can be used in the patch include without being so limited, salcaprozate sodium (SNAC); fatty acids and derivatives, such as sodium caprate, sodium caprylate, and palmitoylcarnitine; bile salts and other steroidal detergents, such as deoxycholate, sodium taurocholate (NaTaC), glycocholate, and saponin; natural and synthetic surfactants, such as phospholipids, sodium lauryl sulfate, dioctyl sodium sulfosuccinate, and derivatives thereof; chelators such as EDTA, citric acid/citrate, and polyacrylates; positively charged polymers such as chitosan; cyclodextrins and derivatives; laurocapram and derivatives; thiomenthol and derivatives; and polysorbates and derivatives. In a specific embodiment, the permeation enhancer is NaTaC. Without being so limited, about 0.5 mg - 50 mg of permeation enhancer could be included in the mucosal suction patch.

Without being so limited, excipients such as adjuvants can be loaded inside one or more compartment(s) (cavities/bulbs) of the patch, such as but not limited to squalene and vitamine E (a-tocopherol).

The number of bulbs in the patch that contains a drug formulation ranges from one to all. If the patch includes two or more bulbs including a drug formulation, the two or more drug formulations may be identical or different.

### Methods of use

The present disclosure provides for the use of a mucosal suction patch described herein for the non-parenteral systemic delivery of a drug in a subject in need thereof. The patch is placed on a mucosa for a time sufficient to ensure drug delivery. In specific embodiments, the time is between about 1 minute to about 10 hours (or about 2, 3, 4, 5, 6, 7, 10, 15, 20 minutes to 1, 2, 3, 4, 5, 6, 7, 8 or 9 hours; *e.g.,* 1-5 min; 1-10 min; 1-15 min; 1-30 min; 1 min to 2h; 1 min to 3h; 5-10 min; 5-15 min; 5-30 min; 5-60 min; 5 min to 2h; 10-30 min; 10-60 min; 15-30 min; 15-45 min; 15-60 min; 15-180 min; 30-60 min; 30 min to 2 h, 30 min to 3 h, 1 h to 2 h, 1h to 3 h, 2-5 h; 2-10 h; and 5-10 h, etc.). In other specific embodiments, the time range is between 5 minutes and 3 hours.

As the drug and excipients are encapsulated in the patch, they are not or not much diluted throughout the application time. A steep drug concentration gradient can thus be established and maintained to achieve better drug permeation.

The delivery of the drug from the patch through the mucosa of the subject and into the subject's blood vessels occurs through passive migration of the drug promoted by the mucosa distortion resulting from the suction, and eventually the permeation enhancer. According to the present disclosure, suction and drug delivery are simultaneous, i.e., conducted as a single step. According to the present disclosure, the patch is static (i.e., does not move once placed on the mucosa and throughout drug delivery). This method of delivery advantageously allows the drug to cross the mucosal epithelium, the barrier for absorption, which typically has a thickness between 150 µm and 800 µm in humans. The drug thereby reaches the systemic circulation via lymphatic vessels and/or blood vessels. It is advantageously a needle free, electricity free delivery that does not involve physical piercing of the mucosa.

### Combination therapies

The mucosal suction patch of the present disclosure can be applied as a stand-alone delivery means for drugs or in combination with other drug delivery strategies, such as permeation enhancers (*e.g.,* NaTaC). Thermal treatment can also enhance the permeation of drugs through the mucosa. A heat-generating segment inside or outside the patch could further increase drug permeation.

### Route

The mucosal suction patch can be located on any subject mucosa to deliver a drug. In preferred embodiments, it will be placed in easily accessible (non-invasively) mucosa in a location that does not prevent breathing or food/drink consumption. Without being so limited, the mucosa is an oral (*e.g.,* cheek, palate, labial or sublingual mucosa) or vaginal mucosa.

### Subject

As used herein the term "subject" is meant to refer to any animal, such as a mammal, including a human, dog, cat, pig, cow, monkey, cattle, horse, etc. In a particular embodiment, it refers to a human.

### Patch design and manufacturing method

The patches of the present disclosure can be designed using common Computer Aided Design (CAD) softwares, such as AutoCAD^{™}, Solidworks^{™}, etc. The patches can be produced using any known method, including but no limited to 3D printing with techniques such as digital light processing (DLP), carving, molding (*e.g.,* compression molding, mold casting, and injection molding), which enables rapid prototyping, optimization, as well as mass production. Without being so limited, molds can be produced in stainless steel and aluminium, or 3D printed material and other materials.

### Drug loading

Any known drug loading method can be used including but not limited to film formation (*e.g.,* drug dispersion in a lipid matrix or polymer matrix), paste injection, mini-tablet loading, etc. With the film formation method, the drugs and excipients are dissolved or dispersed in a suitable solvent, which is then evaporated to leave a solid matrix inside the patch. When the the film formation is conducted by drug dispersion in a lipid matrix, the lipidic matrix can be heated, deposited in the patch and cooled down. In the paste injection method, a semi-solid solution or dispersion of the drug and excipients is deposited by extrusion in the patch.

### Drug delivery

Once the drug loaded patch adheres to the mucosa, and the mucosa is deformed so as to partially fill the cavity of the patch due to the negative pressure (suction effect), the drug diffuses in the medium (*e.g.,* drug formulation, sealing fluid, biological fluid or combination of at least two thereof) inside the patch until it reaches the mucosa. Without being so limited, in Examples presented herein, the negative pressure created by the patch enabled the drug to reach *ex-vivo* a depth of about 500-4500 µm in the animal tissue, thereby crossing the mucosa epithelium (thickness of about 150-800 µm in humans). The drug crosses the mucosal layers, and reaches the blood and/or lymphatic vessels (systemic circulation).

### Non exhaustive advantages

Non-invasive technology. *In vivo* experiments on beagle dogs have demonstrated the minimally invasive nature of this method (FIGs. 10A-C). The reversible deformation of the mucosa does not severely compromise the structural integrity of the mucosa surface, in contrast to techniques such as microneedles which pierce the mucosa. The non-invasiveness is supported by *ex-vivo* investigations that show the integrity of the mucosa layer (FIGs. 4A-B).

Patient friendliness and reduced health care cost. In addition to being non-invasive, the patch application is simple and user friendly as no special training or other devices are required. Therefore, drugs that typically must be administered via injections can be pain-free self-administrated by patients, which most likely increases patient acceptance and hence their compliance, especially for children or subjects with antipathy to needles. The fact that the device is self-administrable also allows the home administration of drugs, which further increases patient comfort as well as reduces the costs and requirements for medical staff.

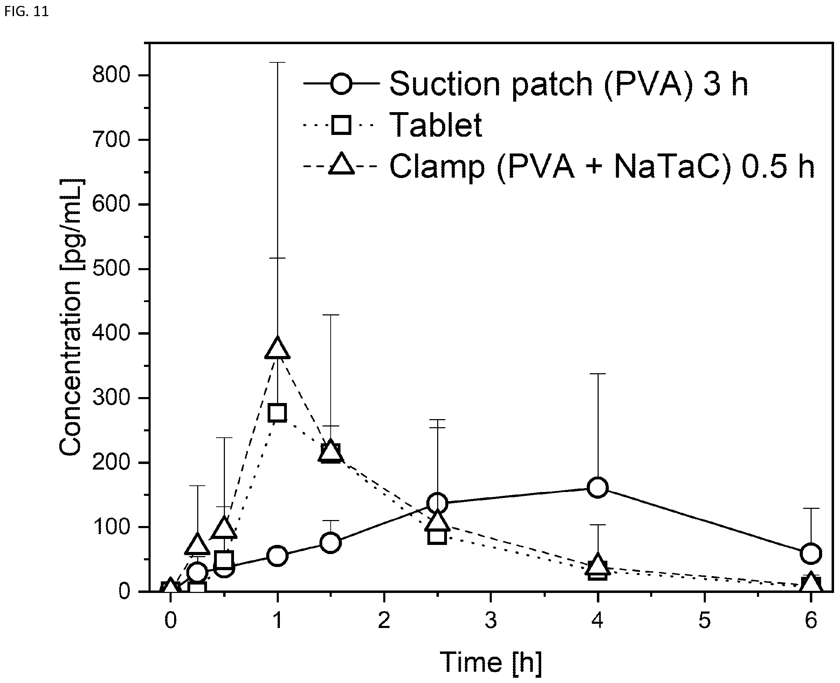

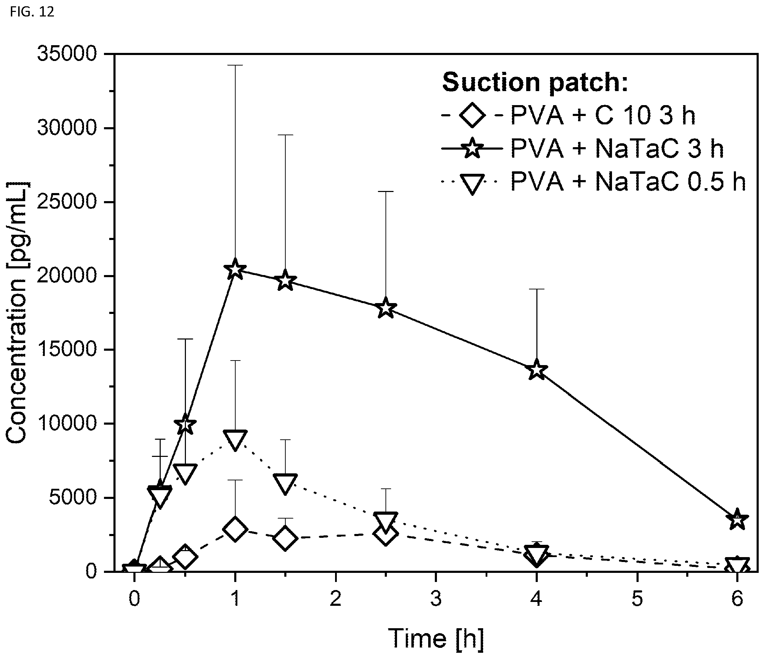

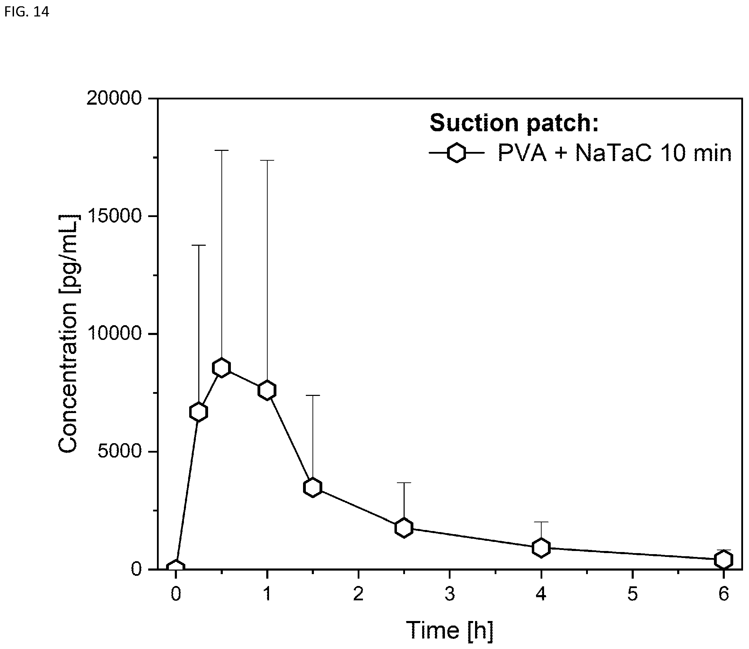

Higher bioavailability. The bioavailability of most oral formulations for peptides is very low, i.e., 1% or lower. Patches bypass hepatic first-pass metabolism and mild chemical and enzymatic environment. The strong hypobaric force of patches of the present disclosure prolonged the retention time of the drug in the vicinity of the epithelium but also disrupted the mucosa barrier integrity, facilitating the permeation of drug molecules. *In vivo* studies on beagle dogs have demonstrated herein that the mucosal suction patch could achieve 35% higher bioavailability than commercial tablets for a model peptide drug, desmopressin (>1100 Da), after 3-hour application time (FIG. 11). When combined with permeation enhancers, the suction patch device could achieve more than 10-times higher oral bioavailability than commercial tablets (FIGs. 12 and 14). A continuous increase in the plasma drug concentration during and even after the suction patch application could be observed.

Versatility. The patch is usable for the delivery of various types of drugs and excipients. The dimension and the shapes of the patches of the present disclosure can be easily adapted according to the patient's need *e.g.,* smaller size for children. The patches can be used for several different dosage forms (*e.g.,* solids or pastes) without major adaptations, several excipients that allow tailoring release kinetics to the drug and several drugs.

Strong and robust mucoadhesiveness without chemical interactions (i.e., without the need for a mucoadhesive coating). In principle, due to the atmospheric pressure, the patch can achieve a 10⁵ Pa (about 100 kPa) adhesion strength without the use of mucoadhesive coating, which is about two orders of magnitude stronger than that of existing mucoadhesive materials.

Scalability and costs. Due to the simple and scalable manufacturing process by molding, pre-existing manufacturing plants can be used with small adjustments. Moreover, the drug loading processes by *e.g.,* film formation or paste injection, are well established processes and can be easily adapted. As a result, simple manufacturing, low cost materials and simple implementation in existing manufacturing plants make the technology readily available for small and large companies. A mold casting process has been established with 3D printed casting molds that allow the production of several patches at a time. A similar casting mold with more patches per plate can be produced by aluminum typically used by industry. As a result, the scalability of the manufacturing process could be established with an FDA approved material that highly simplifies the industrial translation compared to other technologies *e.g.,* microneedles or ultrasound.

When compared to specific technologies that are under clinical trials, patches of the present disclosure present the following advantages. Compared with microneedle based oral capsules (*e.g.,* as developed by Rani Therapeutics): the patch is a much simpler technology involving lower production costs using straightforward manufacturing; the patch is a more robust drug delivery process with lower variability as compared to that obtained in the GI tract with the Rani microneedle based oral capsules due to their complex actuation mechanism; the patch presents fewer risks of pathogen exposure due to its noninvasive nature as compared to those presented by needle puncturing in the GI tract. Compared with aerosol spray based system as developed by Generex Biotechnology which utilizes mechanical pressure to deliver drug compounds through the oral (*e.g.,* buccal) mucosa: the patch can be worn for a continuous period of time, allowing the steady permeation of drug compounds while the pressure generated by the aerosol sprays is transient; by providing tunable drug compartment(s) and various loading strategies, the patch provides better control over the drug dose and versatility with regard to drugs as and drug formulations. Compared with biochemical modifications with permeation carriers as developed by Applied Molecular Transport Inc: no chemical modification of the drug is required with the patch so that no new chemical entities is created, thereby simplifying the drug development and approval process. Finally, since the device (patch) can be conceived without the use of proteinic carrier in the formulation, there is also a reduced risk of generating immune reactions and the associated side effects.

### BRIEF DESCRIPTION OF THE DRAWINGS

In the appended drawings:
FIGs. 1A-C. Example design of a mucosal suction patch based drug delivery system according to the disclosure. (FIG. 1A) An example of 2D structure of the mucosal suction patch including dimensions that have been used for *in vivo* assays presented herein (Type 1, Examples 4 and 6-14). (FIG. 1B) Cross sectional view of the mucosal suction patch structure, loaded with a drug formulation. (FIG. 1C) Final 3D printed poly(β-thioether ester) mucosal suction patch used *in ex vivo* and *in vivo* Examples 4 andd 6-14 compared to a 5-cent coin.
FIGs. 2A-I. Exemplary sketches of different types of mucosal suction patch structures that can be utilized for drug delivery in accordance with the present disclosure. (FIG. 2A) A simple disc-like mucosal suction patch with a drug compartment/bulb (mucosa sealing surface side shown). (FIG. 2B) Hierarchical mucosal suction patch structures with an embedded array of bulbs/cavities, where the drugs can be loaded inside and/or between patch layers. (FIG. 2C) A bio-inspired mucosal suction patch with a large bulb/drug compartment, bottleneck structure and pattern on the contact area on mucosa sealing surface. (FIG. 2D) A similar structure as FIG. 2C but including a wall-spring-like bulb/drug compartment. (FIG. 2E) An example of a multi-layered mucosal suction patch structure with the possibility of incorporating different functionalities, *e.g.,* heating in-between the single layers. (FIG. 2F) An example of mucosal suction patches comprising an array of bulbs and a solid backing layer. The bulbs are connected by the backing layer, which itself contains a drug compartment between the bulbs. (FIG. 2G). An example of a 3D printed mucosal suction patch with 7 individual bulbs and backing layer produced using a biodegradable polymer i.e. poly(β-thioether ester) and compared to a 1 cent coin. (FIG. 2H) An example of a hierarchical mucosal suction patch with an embedded array produced by 3D printing with a biodegradable polymer i.e. poly(β-thioether ester). (FIG. 2I) Type 1' suction patch A, structurally close toType 1 (FIG. 1A/1C) but comprising a cylinder on top to allow the insertion of a floss (Example 1), produced by molding with food-grade polydimethylsiloxane (PDMS).

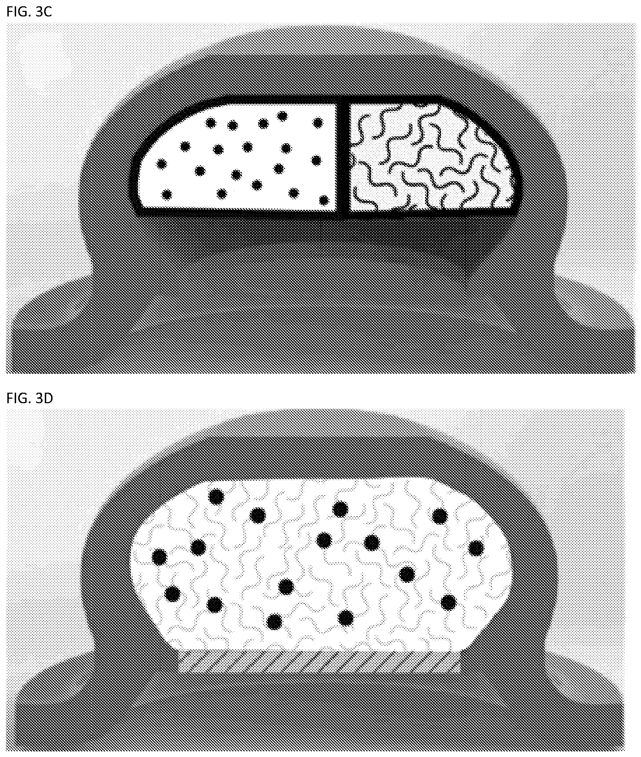

FIGs. 3A-D. Examples of suction patch loading methods for different drug formulations, presented by a vertical cross-sectional view. (FIG. 3A) Multilayer coating: first layer containing the drug/active pharmaceutical ingredient (API) (black dots) and matrix (white) made of polymers and other excipients; second layer (gray, striped) consisting of other polymers and excipients. (FIG. 3B) Single layer coating with matrix (light gray) consisting of a polymer as well as other excipients and the drug/API (black dots). (FIG. 3C) Multiple compartments: several sealed and separated compartments. For example, one side contains a matrix (white) of polymer and other excipients together with the drug/API (black dots); the other side can contain a liquid, hydrogel, or organogel. The separation barrier breaks during the application and compression allowing the two components to mix. (FIG. 3D) Single compartment with a non-solid loading: a drug/API (black dots) can be dissolved or dispersed inside a liquid or semi-solid matrix (gray waves on white background) containing solvents and other excipients. After loading, the formulation is sealed inside the patch by a polymeric film (black stripes on gray background).
FIGs. 4A-B. Tissue integrity and histology. (FIG. 4A) Fluorescence image of 20 µm cryopreserved tissue slice after 3 h mucosal suction patch (Type 1, FIG. 1A/FIG. 1C) application loaded with 54 µg a model surrogate, the dye Cy5 and 1.2 mg PVA. The tissue was stained with iFluor^{™} 488-conjugated phalloidin (F-Actin stain) and Hoechst 33342 (Nucleus stain) and imaged with a widefield microscope (40x). Small single images were obtained from three channels (Ex: 340-380, 460-500, and 590-650 nm, Em: 450-490, 512-542, 662-738 nm, respectively), overlayed and merged. (FIG. 4B) Hematoxylin and eosin (H&E)-stained tissue slice from the same sample.

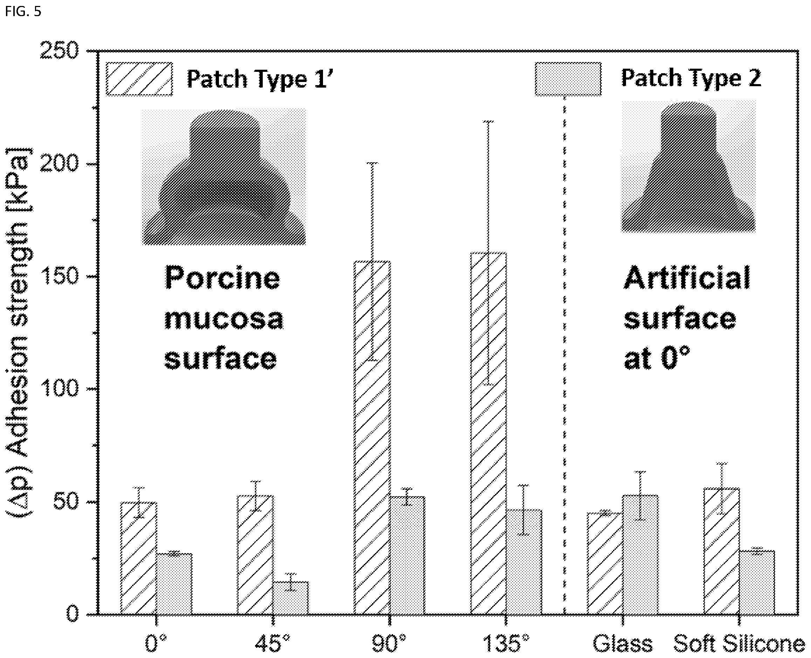

FIG. 5. Adhesion force for different 3D printed suction patch designs (Type 1' and Type 2) produced with abiodegradable polymer poly(β-thioether-ester) and tested on porcine mucosa at different angles (0°, 45°, 90°, and 135°) as well as artificial surfaces. Error bars indicate the standard deviation for n = 9 measurements with m = 3 patches (each measured 3 times).
FIGs. 6A-E. Effect of permeation enhancer in combination with the mucosal suction patch (Type 1, FIG. 1A/FIG. 1C). *Ex vivo* evaluation of drug permeation through the porcine buccal mucosa. (FIG. 6A) Penetration depth profile (normalized to maximum fluorescence intensity) of a model surrogate (Cy5). The mucosal suction patches were loaded with 40 µL of a solution containing 2 mM Cy5 (54 µg), 3% PVA (1.2 mg), and 265 mM various types of permeation enhancers, i.e., sodium taurocholate (NaTaC) (5.7 mg), sodium caprate (C10) (2.1 mg) and salcaprozate sodium (SNAC) (3.2 mg). Data are presented as means (n = 3 samples) + standard deviation (n = 15 measurements). (FIG. 6B) Fluorescent image of the slice of buccal mucosa after applying the mucosal suction patch formulation containing Cy5 and PVA for 3 h. (FIG. 6C) Fluorescent image of the slice of buccal mucosa after applying the mucosal suction patch formulation containing Cy5, PVA and SNAC for 3 h. (FIG. 6D) Fluorescent image of the slice of buccal mucosa after applying the mucosal suction patch formulation containing Cy5, PVA and C10 for 3 h. (FIG. 6E) Fluorescent image of the slice of buccal mucosa after applying the mucosal suction patch formulation containing Cy5, PVA and NaTaC for 3 h.
FIG. 7. *Ex vivo* evaluation of surrogate permeation through the porcine buccal mucosa at different molecular weights administrated with the mucosal suction patch. The mucosal suction patch was loaded as described in FIG. 6 with (a) 160 µg 2k-PEG-Cy5 (~2 kDa, stars), 207 µg 20k-PEG-Cy5 (~20 kDa, squares), or 304 µg Alexa647-Ovalbumin conjugate (~45 kDa, circles); (b) 5.7 mg NaTaC; and (c) 1.2 mg PVA and applied onto the porcine buccal tissue for 3 h. The specimen was processed as described in Example 6. The penetration depth profile of the surrogates was plotted against the fluorescence intensity (normalized to maximum exposure) in log-scale. Data are presented as means (n = 3 samples) + standard deviation (n = 15 measurements). The graph shows that molecules with different molecular weight of more than 1kDa can permeate ≥500 µm and, thus, cross the mucosal penetration barrier.
FIG. 8. Effect of hypobaric pressure on the diffusion of marker molecule. *Ex vivo* evaluation of the drug permeation through porcine buccal mucosa under controlled pressure. The mucosal suction patch (Type 1, FIGs. 1A/1C) was loaded with 54 µg Cy5, 2.1 mg C10 and 1.2 mg PVA and applied onto the porcine buccal tissue for 1 or 3 h. The specimen was processed as described in Example 6. The penetration depth profile of Cy5 was plotted against the fluorescence intensity (linearly normalized to exposure time and gain) in log-scale. Data are presented as means (n = 3 samples) + standard deviation (n = 15 measurements).

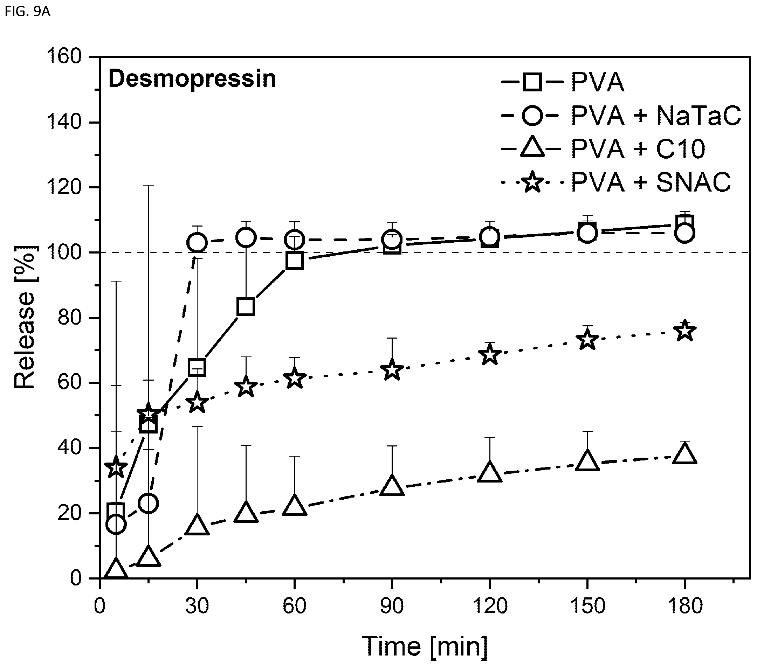

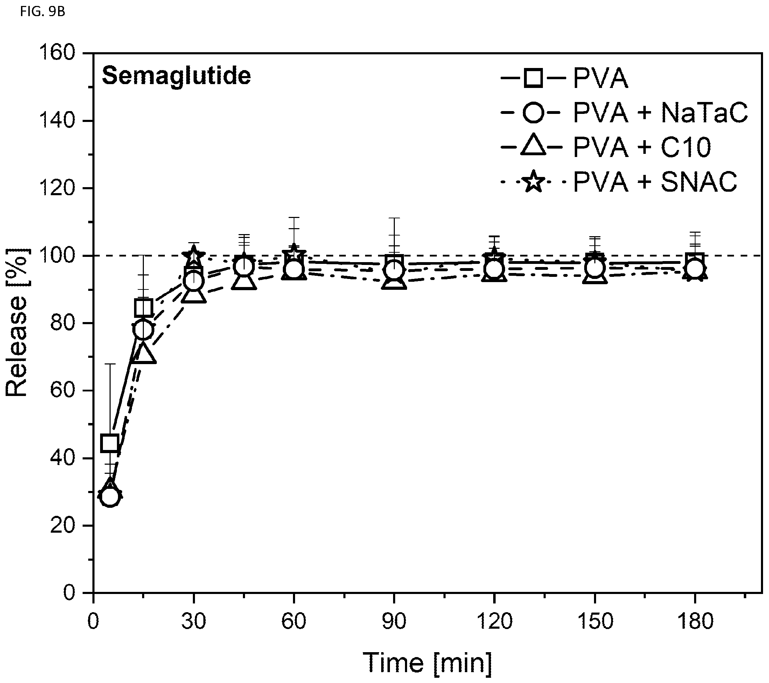

FIGs. 9A-B. *In vitro* release of desmopressin and semaglutide from mucosal suction patch formulations. (FIG. 9A) The mucosal suction patch (Type 1, FIGs. 1A/1C) was loaded with 1.2 mg of desmopressin, 1.2 mg PVA, and either one of various types of permeation enhancers, i.e., 5.7 mg NaTaC (n = 3), 2.1 mg C10 (n = 5) and 3.2 mg SNAC (n = 3). (FIG. 9B) The mucosal suction patch was loaded with 3 mg of semaglutide, 1.2 mg PVA, and either one of various types of permeation enhancers, i.e., 3.9 mg NaTaC (n = 3), 1.4 mg C10 (n = 5) and 2.2 mg SNAC (n = 3). Data are presented as means + standard deviation.

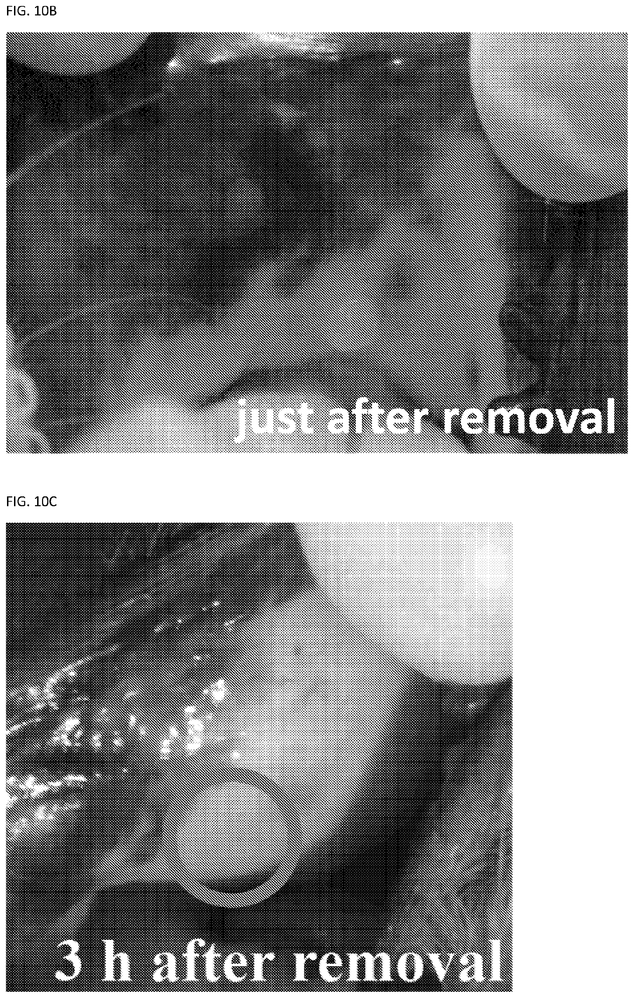

FIGs. 10A-C. *In-vivo* application of mucosal suction patch and risk assessment. *In vivo* application of mucosal suction patch (Type 1, FIGs. 1A/1C) formulation on the buccal mucosa of beagle dogs. (FIG. 10A) Adhesion of a mucosal suction patch on dog buccal mucosa immediately after application. (FIG. 10B) The application site (patch applied for 3 h) of the mucosal suction patch immediately after its removal and (FIG. 10C) the same position 3 h after removal.
FIG. 11. Pharmacokinetic study comparing mucosal suction patch to oral tablets. Plasma concentrations of desmopressin after the oral administration of the tablet or application of the formulations of desmopressin (1.2 mg) with suction (mucosal suction patch (Type 1, FIGs. 1A/1C) (1.2 mg PVA) 3 h) and without suction (Clamp (1.2 mg PVA + 5.7 mg NaTaC) 0.5 h) in beagle dogs. Data are presented as means ± standard deviation (n=3).
FIG. 12. Pharmacokinetics study with desmopressin and different permeation enhancers as well as different application times. Plasma concentrations after applying the mucosal suction patches (Type 1, FIGs. 1A/1C) to beagle dogs, containing desmopressin (1.2 mg), sodium caprate (C10) (2.1 mg) or sodium taurocholate (NaTaC) (5.7 mg) and 1.2 mg PVA. The mucosal suction patches were removed either at 3 h or 0.5 h. Data are presented as means ± standard deviation (n=3).

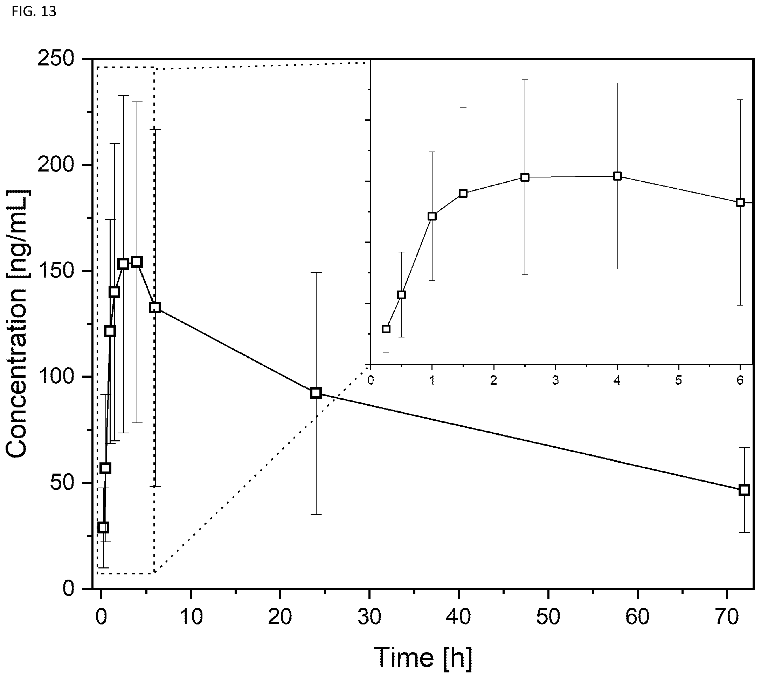

FIG. 13. Pharmacokinetics study with semaglutide (4 kDa). Plasma concentrations after applying the mucosal suction patches (Type 1, FIGs. 1A/1C) containing 9 mg semaglutide, 11.7 mg sodium taurocholate (NaTaC) and 1.2 mg PVA to beagle dogs. The mucosal suction patches were removed at 3 h. The nested graph presents an enlargement of the first 6 h of the study. Data are presented as means ± standard deviation (n=3).
FIG. 14. Pharmacokinetics study with desmopressin with sodium taurocholate (NaTaC) and PVA. Plasma concentrations after applying the mucosal suction patches (Type 1, FIGs. 1A/1C) to beagle dogs, containing desmopressin (1.2 mg), 5.7 mg sodium taurocholate (NaTaC) and 1.2 mg PVA. The mucosal suction patches were removed at 10 minutes. Data are presented as means ± standard deviation (n=3).

### DESCRIPTION OF ILLUSTRATIVE EMBODIMENTS

The present disclosure is illustrated in further details by the following non-limiting examples.

### EXAMPLE 1: Mucosa patch preparation

### Mucosal patch design

The mucosal suction patch was designed with 3D Computer Aided Design (CAD) software SolidWorks^{™}. An example design of the mucosal suction patch that has been used for *in vivo* studies has an outer diameter from 5 mm to 3 cm and a height from 1 mm to 1 cm. The dimension of the device can be easily adjusted according to the drug dose, formulation as well as patient physiology.

### 3D printing

To manufacture the mucosal suction patches (Type 1, 1' and 2) used in Examples 4-14, poly(β-thioether ester) polymer (14 g), the initiator phenylbis(2,4,6-trimethylbenzoyl)phosphine oxide (BAPO) (0.75 wt%), the photo absorber Sudan I (0.04 wt%), the dilution solvent N-Vinylpyrrolidone (NVP)) (11 wt%), and UV inhibitor vitamin E (0.3 wt%) were mixed and sonicated at 80 °C until a homogeneous resin was obtained. A commercial DLP 3D printer (Asiga PICO2, Sydney, Australia) was employed to build the 3D objects. The printer comprised a LED light source of 405 nm and a customized tray with a heating system to allow printing at various temperatures *e.g.,* 75 °C. After printing, resin residuals were removed with isopropanol and ethanol. Subsequently, the mucosal suction patch was cured under 405 nm UV-light for 15 min. See FIGs. 1A-C.

### Molding

Type 1 suction patch structure was modified in CAD software SolidWorks^{™} by adding a small cylinder (Ø 3 mm, height 2 mm) on top of the patch's dome to allow the insertion of a floss and thus design the Type 1' patch (FIG. 2I). The designs were inverted and split in half to prepare negative molds, with multiple entities, allowing for an upscale production. The molds consisted of two parts (i.e. main body and insert).

To manufacture the suction patches, the food-grade polydimethylsiloxane (PDMS) (Bluesil RTV 3428, Elkem) base and the curing agent were mixed at a 10 to 1 ratio (w/w), followed by vacuum degassing for 5 min to remove all air bubbles. Then, the PDMS pre-polymer was poured onto the mold and vacuum-degassed for another 5 - 10 min. Subsequently, the insert was placed onto the main body and the combined plates were placed in a holder to uniformly apply pressure during the PDMS hardening. Specifically, PDMS was cured overnight at room temperature. This process was always carried out with two molds simultaneously. Afterwards, the insert and the excess material were removed. Two similar and clean main body molds were coated with a thin layer of freshly prepared and degassed PDMS pre-polymer and merged. Similar to the previous step, the combined molds were placed into the holder, closed, and allowed to cure overnight. Finally, the combined molds were opened, the suction patches unmolded and the excess material removed.

### EXAMPLE 2: Variety of 3D structures of mucosal suction patches

The mucosal suction patch drug delivery device can have various 3D shapes. Without being so limited, it includes single bulb patch with tunable drug compartment sizes; multiple bulbs with a backing layer (*e.g.,* FIG. 2F); plurality/array of bulbs nested inside a larger bulb (*e.g.,* FIG. 2B); mucosal suction patches with patterned mucosa sealing surface (*e.g.,* FIG. 2C); multilayer patch structures in which a heat-generating material/device could be incorporated (*e.g.,* FIG. 2E). Designs can be directly produced by various manufacturing methods such as 3D printing and molding. See FIGs. 2A-I.

### EXAMPLE 3: Mucosal suction patch drug and surrogate/fluorescent dye loading

### Drug surrogates/fluorescent dyes

A stock buffer solution was prepared by first dissolving 15k PVA in deionized (DI)-water to a final concentration of 3% w/v.

The drug surrogate Cy5 (54 µg/2 mM Cy5 in Examples 4, 6 and 8; 160 µg/2mM 2k-PEG-Cy5 in Example 7; and 207 µg/258 µM 20k-PEG-Cy5 in Example 7); or Alexa647 (304 µg/168 µM Alexa647-Ovalbumin conjugate in Example 7) was added to the stock solution together with a permeation enhancer (5.7 mg/ 265 mM NaTaC in Example 6 or 7; 2.1 mg/ 265 mM C10 in Examples 6 and 8; or 3.2 mg/ 265 mM SNAC in Example 6) to form a clear solution.

The mucosal suction patches (Type 1, FIG. 1A/FIG. 1C) were loaded by drop-casting 40 µL of the fresh clear solution inside the mucosal suction patch cavity and allowed it to dry overnight in ambient conditions and subsequently 2 - 4 h under vacuum (loading schematically illustrated in FIG. 3B). Each mucosal suction patch was thus loaded with a surrogate and 1.2 mg PVA (loading schematically illustrated in FIG. 3B).

### Desmopressin

A stock buffer solution was prepared by first dissolving 15k PVA in DI-water to a final concentration of 3% w/v.

Desmopressin (26.56 mM (Examples 9-12, and 14)) was added to the stock solution, together with a permeation enhancer with a concentration of 265 mM (5.7 mg NaTaC in in Examples 9, 11-12 and 14; or 2.1 mg C10 in Examples 9-10 and 12), to form a clear solution.

The mucosal suction patches (Type 1, FIG. 1A/FIG. 1C) were loaded by drop-casting 40 µL of the fresh clear solution inside the mucosal suction patch cavity and allowed to dry overnight in ambient conditions and subsequently 2 - 4 h under vacuum. Each mucosal suction patch was thus loaded with 1.2 mg desmopressin and 1.2 mg PVA (loading schematically illustrated in FIG. 3B).

### Semaglutide

A stock buffer solution was prepared by first dissolving 15k PVA in Tris buffer to a final concentration of 3% w/v in example 9 or 1.5% w/v in Example 13.

Semaglutide (18.23 mM (Example 9), 27.35 mM (Example 13)) was added to the stock solution, together with a permeation enhancer with a concentration of 182.3 mM (3.9 mg NaTaC, 1.4 mg C10 or 2.2 mg SNAC in Example 9), or 273.5 mM (11.7 mg NaTaC in Example 13), to form a clear solution.

The mucosal suction patches (Type 1, FIG. 1A/FIG. 1C) were loaded by drop-casting 40 µL (Example 9) or 80 µL (Example 13) of the fresh clear solution inside the mucosal suction patch cavity and allowed to dry overnight in ambient conditions and subsequently 2 - 4 h under vacuum. Each mucosal suction patch was thus loaded with 3 mg of semaglutide (Example 9) or 9 mg of semaglutide (Example 13) and 1.2 mg PVA (loading schematically illustrated in FIG. 3B).

### EXAMPLE 4: Ex vivo evaluation of surrogate (fluorescent dye) loaded mucosal suction patch device on fresh porcine buccal mucosa

*Ex vivo* evaluation of the surrogate (fluorescent dye) loaded mucosal suction patch device (Type 1, FIG. 1A/FIG. 1C) was performed on fresh porcine buccal mucosa tissue obtained from a local slaughterhouse. The entire cheek was harvested and placed immediately after slaughter in a buffer, *e.g.,* DMEM/F-12 (Dulbecco's Modified Eagle Medium/Nutrient Mixture F-12), HEPES (4-(2-hydroxyethyl)-1-piperazineethanesulfonic acid), without phenol red, pH 7.4, 1 v/v% antibiotic antimycotic (10000 units penicillin, 10 mg streptomycin and 25 µg Amphotericin B per mL).

The tissue was transported on ice to the laboratory, where the underlying tissue was removed with a scalpel and surgical scissors to a thickness of ~ 1-2 cm. After cutting, the tissue was washed with PBS and placed in a 6 well plate with fresh buffer solution and incubated for 20 min at 37 °C. For the mucosal suction patch application, the tissue specimens were removed from the buffer for max. 3 min. Afterwards, 80 µL deionized water was added inside the prepared mucosal suction patch and applied onto the buccal mucosa by hand within 2 min. Subsequently, the specimens were incubated for 3 h at 37 ± 2 °C in an oven before the mucosal suction patch was removed. The tissue was then washed with PBS to remove excess marker molecules followed by snap-frozen using liquid nitrogen chilled isopentane for 30 sec. Afterwards, excess tissue was removed with a razor blade and 20 µm thick slices on glass slides were then obtained using a cryotome at - 20 °C. After warming up to room temperature for 30 min, the glass slides were fixed for 5 min in methanol-free para-formaldehyde PBS solution (pH 7.4) and washed for 3 min.

Samples for fluorescence imaging are shown in FIG. 4A. Specimen were permeabilized with a 0.5 % Trition^{™} X100 PBS buffered solution for 15 min, washed for 3 min in PBS and incubated with a PBS buffered solution of 0.1 % iFluor^{™} 488-conjugated phalloidin (F-Actin stain) and 0.1 % Hoechst 33342 (Nucleous stain) for 30 min. Finally, the slides were washed for 3 min in PBS and mounted with a ProLong^{™} Diamond (Thermo Fisher) mounting medium and a coverslip. In contrast, samples for quantification, as described in Examples 6-8, were mounted directly after fixation and washing for 3 min in PBS as described above.

Finally, H&E staining (FIG. 4B) was carried out by an automated H&E stainer with each step 90 sec staining and 3 sec dripping. The slides were immersed in 100 % ethanol (EtOH), 100 % EtOH, 95 % EtOH, 70 % EtOH, DI-H₂O, Mayer's hematoxylin, Mayer's hematoxylin, tap water, HCl-EtOH (70% EtOH, 29.9 % dd H₂O, 0.1 % hydrochloric acid (37%)), tap water, dd H₂O, Eosin Y, Eosin Y, 95 % EtOH, 95 % EtOH, 100 % EtOH, 100 % EtOH, xylene, xylene, and finally mounted with a coverslip and Entellan for imaging with a widefield microscope. While physically deformed (stretched), the integrity of the mucosal structures was preserved. Results are shown in FIGs. 4A-B.

### EXAMPLE 5: Evaluation of the patch's adhesive properties

The adhesive properties of the mucosal suction patch (Types 1' and 2) were evaluated using a texture analyzer (TA.XT plus Texture Analyser, 50 kg load cell, Stable Micro Systems, Godalming (UK)). Adhesion tests were performed by gluing porcine mucosa tissue, soft silicone or a glass substrate (artificial surface) to a hard 3D printed platform with different angles that were mounted with standard clamps. The suction patch was compressed onto the test surface and pulled up vertically to the clamp until it detached (rupture). During pulling at a constant rate, the force and the pulling distance were measured and recorded. The adhesion strength was calculated by dividing the measured adhesion force by the inner diameter (contact area). Results are presented in FIG. 5 and show that the mucosal suction patch provided from 1 N/cm² to 20 N/cm² [≈10 kPa to ≈200kPa] to adhesive strength under various pulling conditions.

### EXAMPLE 6: Ex vivo evaluation of mucosal suction patch impact on drug permeation with Cy5 as surrogate through the porcine buccal mucosa

Fluorescence images were obtained with a widefield microscope (Leica DMI 6000) at 20x magnification in the tile scan mode and merged to a single image. The specimen was excited at 590-650 nm and the emission filtered from 662-738 nm. To evaluate the penetration depth of the surrogate, images were processed as grayscale with Fiji-ImageJ^{™} software. Line profiles of the grayscale intensity were obtained by drawing multiple straight lines vertical to the surface of the mucosa lining. The obtained intensity-distance profiles were transferred to Microsoft Excel^{™} and MatLab^{™} for further processing. The autofluorescence was first corrected by subtracting the averaged baseline intensity multiplied by 3 times its standard deviation obtained from a lower laying region of the tissue. The maximum intensity was set to 100% and the corresponding distance value was set to zero to normalize the drug distribution profile. As a result, the penetration depth profile of the surrogate could then be plotted and compared among different conditions or formulations. Results are presented in FIGs. 6A-E and show the performance of the permeation enhancers depending on the drug.

### EXAMPLE 7: Ex vivo evaluation of permeation of surrogates of different molecular weights (~ 2 to 45 kDa) through mucosa when administrated with mucosal suction patch

The molecular weight range of administrable drugs was evaluated *ex-vivo* on porcine buccal mucosa tissue by applying drug surrogates with different molecular weights. Samples were prepared as described in Example 3 and processed as described in Example 6 with n = 3 samples with n = 15 measurements. The following surrogates and formulations were used: 2k-PEG-Cy5 and 20k-PEG-Cy5 are Cy5-dye linked to polyethylene glycol (PEG) with a molecular weight of 2kDa or 20kDa PEG, respectively. Alexa647-Ovalbumin is the Alexa647 dye linked to ovalbumin (≈ 5.5 nm, 45 kDa). Amount deposited on the mucosa: 2k = 160 µg; 20k-PEG-Cy5 = 207 µg; Alexa647-Ovalbumin = 304 µg. The formulation was the drug surrogate with 1.2 mg PVA and 5.7 mg NaTaC. Results are presented in FIG. 7 and show that all drug surrogates could diffuse at least 500 µm deep inside the tissue and thus cross (2k-PEG-Cy5 and 20k-PEG-Cy5) or reach deep inside (Alexa647-Ovalbumin) the mucosal epithelium. This shows that molecules up to 45 kDa (> 5 nm) or potentially even larger can be administered with the mucosal suction patch and reach the systemic circulation. As a result, large biopharmaceuticals such as recombinant human growth hormone (22.1 kDa) can be administered with the mucosal suction patch.

### EXAMPLE 8: Ex vivo evaluation of hypobaric pressure on the diffusion of marker molecule in porcine buccal mucosa through the mucosal suction patch

To evaluate the effects of hypobaric pressure on the drug permeability, a small electric vacuum pump system was connected to the mucosal suction patch via tubing that connects through a puncture at the top of the patch. The device could continuously monitor and control the pressure inside the patch to a constant value, *e.g.,* 30 kPa. For the application, the buccal tissue was prepared as described above and placed in a 6-well plate that is filled with DMEM buffer. The mucosal suction patch pre-loaded with 54 µg Cy5, 2.1 mg C10 and 1.2 mg PVA in accordance with Example 3, was filled with 80 µL deionized water and placed onto the tissue. Subsequently, the pressure was set by the reduction valve to 30 kPa and left in the 37 °C incubators for 3 h. Afterwards, the vacuum pump was turned off and the mucosal suction patches were detached. The tissue specimen was processed as described before for fluorescent imaging. The intensity of each image was converted to the equivalent of 150 ms exposure time to allow the comparability. When plotting the fluorescent intensity-distance profile, the absolute intensity was utilized instead of the normalized in order to compare the pressure-related permeation enhancing effects among different samples. Results are presented in FIG. 8 and show that the negative pressure and associated deformation of the mucosa effectively enhanced the drug permeation.

### EXAMPLE 9: In vitro release of desmopressin and semaglutide from mucosal suction patch formulations

*In vitro* release studies were performed in 50 mL Falcon tubes containing a 3D printed inlet in order to avoid the mucosal suction patch sinking inside the tip. Mucosal suction patches (Type 1 shown in FIG. 1A/FIG. 1C) containing a formulation of 1.2 mg desmopressin mixed with permeation enhancer NaTac (5.7 mg), C10 (2.1 mg) or SNAC (3.2 mg) and 1.2 mg PVA (FIG. 9A) or of 3 mg semaglutide mixed with permeation enhancer NaTac (3.9 mg), C10 (1.4 mg) or SNAC (2.2 mg) and 1.2 mg PVA were used. The mucosal suction patches were first drug-loaded as described in Example 3 and placed inside 40 mL of an aqueous PBS buffer solution (pH 7.4) for desmopressin or a 15 ml aqueous Tris buffer solution (pH 7.4) for semaglutide.

The temperature was set at 37 °C, and the tubes were shaken at 200 rpm. Aliquots of 1 mL were sampled at 5, 15, 30, 45, 60, 90, 120, 150, 180 min after immersing the mucosal suction patch in the solution. The sampled volume was replaced with the same fresh PBS buffer for desmopressin or the same Tris buffer for semaglutide. Quantification was carried by HPLC (VWR Hitachi Chromaster^{™} system with 5160 pump, 5260 autosampler, 5310 column oven and 5430 diode array detector, VWR International, Radnor, PA) with the UV wavelength set at 220 or 280 nm. The C18 column (XBridge^{™}, 5 µm, 250 x 4.6 mm, Waters, Milford, MA) was used for the separation, while a mixture of 75 % deionized water (0.1 % v/v trifluoroacetic acid) and 25 % acetonitrile was used for the mobile phase. The release study for semaglutide was carried out in Tris buffer, while the remaining set up was the same as that for desmopressin. As a mobile phase, a mixture of 57 % deionized water (0.1 % v/v ortho-phosphoric acid) and 43 % acetonitrile was used for the elution of semaglutide. Results are presented in FIGs. 9A-B and show that the drug release kinetics from the mucosal suction patch depends on the solubility of the drugs as well as the combination with permeation enhancers and other excipients. By tuning the permeation enhancers, polymers and other excipients, it is possible to adjust the full drug release from 1 min to 10 h.

### EXAMPLE 10: In-vivo application of mucosal suction patch and risk assessment

*In-vivo* experiments were conducted at Institute national de la recherche scientifique (INRS) (CNBE, Laval, QC, Canada) and received approval from the ethical committee. Mucosal suction patches were prepared as described in Example 3 and loaded with 1.2 mg desmopressin (D), 2.1 mg sodium caprate (C10) and 1.2 mg PVA. For the study, three beagle dogs with body weights around 10 kg were used. During the whole mucosal suction patch application, images were taken at different time points, i.e., 0 h, 3 h, and 24 h. Results are presented in FIGs. 10A-C and show that the mucosal suction patch reversibly deforms the mucosa.

### EXAMPLE 11: Pharmacokinetic study comparing mucosal suction patch to oral tablets

*In vivo* experiments were performed on the mucosal suction patch (Type 1, FIG. 1A/FIG. 1C) formulation containing 1.2 mg desmopressin and 1.2 mg PVA (without any permeation enhancer) as described in Example 3. As a control formulation, enteric-coated (Eudragit^{®} L100-55, Essen, Germany) capsules containing commercial Minirin^{®} drug tablets (6 pieces containing 1.2 mg desmopressin in total) was given orally to the dog.

To evaluate the effect of adhesion strength on the drug permeation, a control study was performed without compressing the mucosal suction patch. A 3D printed clamp containing a cavity for the suction patch containing 1.2 mg desmopressin, 1.2 mg PVA, and 5.7 mg NaTaC was attached to the dog's mucosa, ensuring similar conditions (i.e., enclosed environment, close contact to the mucosa, same sample preparation and loading) as the applied suction patch, but with permeation enhancer and without negative pressure and the resulting tissue deformation.

The dogs were anesthetized for the duration of the experiment (3 h). The suction remained firmly in place for 3 h and blood samples were withdrawn at predetermined time points, i.e., 0, 15, 30, 60, 90, 150, 240, and 360 min. Blood samples were collected in K₂EDTA tubes immediately followed by centrifugation at 1700 G for 10 min at 4°C. Subsequently, the plasma was stored in clear polypropylene vials at -80°C until further analysis. The drug plasma concentration was evaluated using LC-MS/MS method. Briefly, an aliquot of 900 µL dog plasma sample was mixed with 10 µL internal standard (Goserelin; 25 ng/mL) and 300 µL 4% H₃PO₄ acidified water into 1.5 mL Eppendorf tubes. The plasma sample was then extracted using Oasis^{™} WAX solid-phase extraction cartridges. After extraction, the solution was evaporated under a low vacuum and reconstituted in 120 µL of 11:11:78 acetonitrile/methanol/deionized-water and injected into the Xevo^{™} G2-XS Accurate Mass Spectrometer. Samples were analyzed in TOF-MRM mode with a quantification limit of < 15 pg/mL. Results are presented in FIG. 11 and show that a bioavailability comparable to that of commercial oral tablets could be achieved with the mucosal suction patch alone (i.e., without the addition of a permeation enhancer). A similar bioavailability could also be achieved with permeation alone and no negative pressure.

### EXAMPLE 12: Pharmacokinetics study of mucosal suction patch with permeation enhancers in dogs

*In vivo* experiments were performed on the mucosal suction patch formulation (Type 1, FIG. 1A/FIG. 1C) containing 1.2 mg desmopressin, 1.2 mg PVA, and 2.1 mg sodium caprate (C10) or 5.7 mg sodium taurocholate (NaTaC) as described in Example 3. The patch was applied for 0.5 h or 3 h. The blood sampling time points and the analysis procedure are the same as described in Example 11. Results are presented in FIG. 12 and show that the combination of permeation enhancers and mucosal suction patch significantly increased the bioavailability of the drug compared to that achieved with the desmopressin tablet in FIG. 11, namely in the order of 10 times.

### EXAMPLE 13: Pharmacokinetic study comparing mucosal suction patch of semaglutide

*In vivo* experiments were performed on the mucosal suction patch (Type 1, FIG. 1A/FIG. 1C) formulation containing 9 mg semaglutide and 1.2 mg PVA and 11.7 mg NaTaC prepared as described in Example 3. The dogs were anesthetized for the duration of the experiment (30 min). The suction remained firmly in place for 30 min and blood samples were withdrawn via at predetermined time points, i.e., 0, 15, 30, 60, 90, 150, 240, 360, 1440 (24 h) and 4320 (72 h) min. Blood samples were collected in K₂EDTA tubes immediately followed by centrifugation at 1700 G for 10 min at 4 °C. Subsequently, the plasma was stored in clear polypropylene vials at -80 °C until further analysis. The drug plasma concentration was evaluated using an LC-MS/MS method as described in Example 11. Results are presented in FIG. 13 and show that the mucosal suction patch and permeation enhancer achieved therapeutic relevant plasma concentrations of semaglutide (4 kDa).

### EXAMPLE 14: Pharmacokinetics study of mucosal suction patch with permeation enhancers in dogs

*In vivo* experiments were performed on the mucosal suction patch formulation (Type 1, FIG. 1A/FIG. 1C) containing 1.2 mg desmopressin, 1.2 mg PVA, and 5.7 mg sodium taurocholate (NaTaC) as described in Example 3. The patch was applied for 10 min. The blood sampling time points and the analysis procedure are the same as described in Example 11. Results are presented in FIG. 14 and show that the combination of permeation enhancers and mucosal suction patch significantly increased the bioavailability of the drug compared to that achieved with the desmopressin tablet in FIG. 11, namely in the order of 25 times.

The foregoing results show that the mucosal suction patch can be used as a platform for various drugs.

## Claims

1. A mucosal suction patch having an external volume of between 50 µL and 5000 µL, the patch comprising
at least one bulb forming at least one cavity, each of the at least one cavity being in fluid communication with at least one opening, and one or more of the at least one cavity enclosing a drug;
a mucosa sealing surface delimiting the at least one opening; and
a pressure applying surface opposite to the mucosa sealing surface;
the at least one bulb having an elasticity such that, the at least one bulb being in an expanded state, upon applying mechanical pressure on the pressure applying surface, the at least one bulb is deformed, and when the mechanical pressure is released while the mucosa sealing surface is on a subject mucosa, the at least one bulb at least partially regains its expanded state, temporarily creating negative pressure inside the at least one cavity, which deforms the mucosa, which reversibly fills up at least part of the at least one bulb cavity, causing the drug to migrate in the mucosa.

2. The mucosal suction patch of claim 1, wherein the at least one bulb's elasticity is of about 0.01 MPa to about 1000 MPa.

3. The mucosal suction patch of any one of claims 1-3, wherein the drug is in a formulation further comprising at least one excipient.

4. The mucosal suction patch of claim 3, wherein the at least one excipient comprises a permeation enhancer.

5. The mucosal suction patch of any one of claims 1-4, comprising a single bulb.

6. The mucosal suction patch of any one of claims 1-5, further comprising a sealer.

7. The mucosal suction patch of any one of claims 1-6, wherein the drug has a molecular weight of about 1 kDa to about 50 kDa.

8. The mucosal suction patch of any one of claims 1-7, for transmucosal administration of the drug to a subject.

9. A mucosal suction patch for use in the transmucosal administration of a drug to a subject comprising:
applying mechanical pressure on the mucosal suction patch containing the drug prior to or while placing a mucosal sealing surface of the mucosal suction patch on a mucosa of the subject; and
releasing the mechanical pressure on the mucosal suction patch to create a suction and deformation of the mucosa,
whereby the drug migrates through the mucosal epithelium and enters systemic circulation of the subject.

10. The mucosal suction patch for use of claim 9, wherein the mucosal suction patch adheres to the mucosa with a strength of about 0.5 kPa to about 200 kPa.

11. The mucosal suction patch for use of claim 9 or 10, wherein the patch comprises:
at least one bulb forming at least one cavity, each of the at least one cavity being in fluid communication with at least one opening, and one or more of the at least one cavity enclosing the drug;
the mucosa sealing surface delimiting the at least one opening; and
a pressure applying surface opposite to the mucosa sealing surface.
whereby the at least one bulb having an elasticity such that, the at least one bulb being in an expanded state, upon applying the mechanical pressure on the pressure applying surface, the at least one bulb is deformed, and when the mechanical pressure is released, the at least one bulb at least partially regains its expanded state.

12. The mucosal suction patch for use of any one of claims 9-11, wherein (a) the mucosal suction patch is applied on the mucosa for 1 minute to 10 hours, preferably 5 minutes to 30 minutes; and/or (b) the drug is in a formulation further comprising at least one excipient, preferably the at least one excipient comprises a permeation enhancer.

13. The mucosal suction patch for use of any one of claims 9-12, wherein the mucosa is oral or vaginal mucosa, preferably the mucosa is oral mucosa.

14. The mucosal suction patch for use of any one of claims 9-13, wherein the drug has a molecular weight of about 1 kDa to about 50 kDa.

15. The mucosal suction patch of any one of claims 1-7, wherein the mucosa is oral or vaginal mucosa, preferably wherein the mucosa is oral mucosa.

## Patentansprüche

1. Schleimhautsaugpflaster mit einem Außenvolumen zwischen 50 µL und 5000 µL, wobei das Pflaster Folgendes umfasst
mindestens einen Bulbus, der mindestens einen Hohlraum bildet, wobei jeder der mindestens einen Hohlräume in Fluidverbindung mit mindestens einer Öffnung steht, und einer oder mehrere der mindestens einen Hohlräume ein Arzneimittel einschließen;
eine Schleimhautabdichtungsfläche, die die mindestens eine Öffnung begrenzt, und
eine der Schleimhautabdichtungsfläche gegenüberliegende Druckausübungsfläche;
wobei der mindestens eine Bulbus eine derartige Elastizität aufweist, dass, wenn sich der mindestens eine Bulbus in einem aufgeweiteten Zustand befindet, der mindestens eine Bulbus beim Ausüben von mechanischem Druck auf die Druckausübungsfläche verformt wird, und wenn der mechanische Druck aufgehoben wird, während sich die Schleimhautabdichtungsfläche auf einer zu behandelnden Schleimhaut befindet, der mindestens eine Bulbus zumindest teilweise wieder in seinen aufgeweiteten Zustand zurückkehrt, wodurch vorübergehend ein Unterdruck im Inneren des mindestens einen Hohlraums erzeugt wird, der die Schleimhaut verformt, die reversibel zumindest einen Teil des mindestens einen Bulbushohlraums auffüllt, wodurch das Arzneimittel in die Schleimhaut migriert.

2. Schleimhautsaugpflaster nach Anspruch 1, wobei die Elastizität des mindestens einen Bulbus etwa 0,01 MPa bis etwa 1000 MPa beträgt.

3. Schleimhautsaugpflaster nach einem der Ansprüche 1 bis 3, wobei das Arzneimittel in einer Formulierung vorliegt, die ferner mindestens einen Hilfsstoff umfasst.

4. Schleimhautsaugpflaster nach Anspruch 3, wobei der mindestens eine Hilfsstoff einen Permeationsverstärker umfasst.

5. Schleimhautsaugpflaster nach einem der Ansprüche 1 bis 4, das einen einzelnen Bulbus umfasst.

6. Schleimhautsaugpflaster nach einem der Ansprüche 1 bis 5, das ferner einen Dichtungsstoff umfasst.

7. Schleimhautsaugpflaster nach einem der Ansprüche 1 bis 6, wobei das Arzneimittel ein Molekulargewicht von etwa 1 kDa bis etwa 50 kDa aufweist.

8. Schleimhautsaugpflaster nach einem der Ansprüche 1 bis 7 zur transmukosalen Verabreichung des Arzneimittels an einen Patienten.

9. Schleimhautsaugpflaster zur Verwendung bei der transmukosalen Verabreichung eines Arzneimittels an einen Patienten, umfassend:
Ausüben von mechanischem Druck auf das Schleimhautsaugpflaster, das das Arzneimittel enthält, bevor oder während eine Schleimhautabdichtungsfläche des Schleimhautsaugpflasters auf einer Schleimhaut des Patienten angebracht wird; und
Aufheben des mechanischen Drucks auf das Schleimhautsaugpflaster, um eine Saugwirkung und eine Verformung der Schleimhaut zu erzeugen,
wodurch das Arzneimittel durch das Schleimhautepithel migriert und in den systemischen Kreislauf des Patienten gelangt.

10. Schleimhautsaugpflaster zur Verwendung nach Anspruch 9, wobei das Schleimhautsaugpflaster mit einer Stärke von etwa 0,5 kPa bis etwa 200 kPa an der Schleimhaut haftet.

11. Schleimhautsaugpflaster zur Verwendung nach Anspruch 9 oder 10, wobei das Pflaster umfasst:
mindestens einen Bulbus, der mindestens einen Hohlraum bildet, wobei jeder der mindestens einen Hohlräume in Fluidverbindung mit mindestens einer Öffnung steht, und einer oder mehrere der mindestens einen Hohlräume das Arzneimittel einschließen;
die Schleimhautabdichtungsfläche, die die mindestens eine Öffnung begrenzt; und
eine der Schleimhautabdichtungsfläche gegenüberliegende Druckausübungsfläche;
wobei der mindestens eine Bulbus eine derartige Elastizität aufweist, dass, wenn sich der mindestens eine Bulbus in einem aufgeweiteten Zustand befindet, der mindestens eine Bulbus beim Ausüben des mechanischen Drucks auf die Druckausübungsfläche verformt wird, und wenn der mechanische Druck aufgehoben wird, der mindestens eine Bulbus zumindest teilweise wieder seinen aufgeweiteten Zustand zurückkehrt.

12. Schleimhautsaugpflaster zur Verwendung nach einem der Ansprüche 9 bis 11, wobei (a) das Schleimhautsaugpflaster für 1 Minute bis 10 Stunden, vorzugsweise 5 Minuten bis 30 Minuten, auf die Schleimhaut aufgebracht wird; und/oder (b) das Arzneimittel in einer Formulierung vorliegt, die ferner mindestens einen Hilfsstoff umfasst, wobei der mindestens eine Hilfsstoff vorzugsweise einen Permeationsverstärker umfasst.

13. Schleimhautsaugpflaster zur Verwendung nach einem der Ansprüche 9 bis 12, wobei die Schleimhaut Mund- oder Vaginalschleimhaut ist, vorzugsweise ist die Schleimhaut Mundschleimhaut.

14. Schleimhautsaugpflaster zur Verwendung nach einem der Ansprüche 9 bis 13, wobei das Arzneimittel ein Molekulargewicht von etwa 1 kDa bis etwa 50 kDa aufweist.

15. Schleimhautsaugpflaster nach einem der Ansprüche 1 bis 7, wobei die Schleimhaut Mund- oder Vaginalschleimhaut ist, vorzugsweise ist die Schleimhaut Mundschleimhaut.

## Revendications

1. Patch de succion pour muqueuse ayant un volume externe compris entre 50 µL et 5000 µL, le patch comprenant :
au moins un bulbe formant au moins une cavité, chacune des au moins une cavité étant en communication fluidique avec au moins une ouverture, et une ou plusieurs des au moins une cavité renfermant un médicament ;
une surface d'étanchéité muqeuse délimitant la au moins une ouverture ; et
une surface d'application de pression opposée à la surface d'étanchéité muqueuse ;
le au moins un bulbe ayant une élasticité telle que, le au moins un bulbe étant dans un état expansé, lors de l'application d'une pression mécanique sur la surface d'application de pression, le au moins un bulbe se déforme, et lorsque la pression mécanique est relâchée alors que la surface d'étanchéité muqueuse se trouve sur une muqueuse d'un sujet, le au moins un bulbe retrouve au moins en partie son état expansé, créant temporairement une pression négative à l'intérieur de la au moins une cavité, qui déforme la muqueuse, qui remplit de manière réversible au moins une partie de la au moins une cavité du bulbe, provoquant la migration du médicament dans la muqueuse.

2. Le patch de succion pour muqueuse selon la revendication 1, dans lequel l'élasticité du au moins un bulbe est d'environ 0,01 MPa à environ 1000 MPa.

3. Le patch de succion pour muqueuse selon l'une quelconque des revendications 1 à 3, dans lequel le médicament se trouve dans une formulation comprenant en outre au moins un excipient.

4. Le patch de succion pour muqueuse selon la revendication 3, dans lequel le au moins un excipient comprend un agent favorisant la perméation.

5. Le patch de succion pour muqueuse selon l'une quelconque des revendications 1 à 4, comprenant un seul bulbe.

6. Le patch de succion pour muqueuse selon l'une quelconque des revendications 1 à 5, comprenant en outre un agent d'étanchéité.

7. Le patch de succion pour muqueuse selon l'une quelconque des revendications 1 à 6, dans lequel le médicament a un poids moléculaire d'environ 1 kDa à environ 50 kDa.

8. Le patch de succion pour muqueuse selon l'une quelconque des revendications 1 à 7, pour l'administration transmuqueuse du médicament à un sujet.

9. Patch de succion pour muqueuse pour son utilisation dans l'administration transmuqueuse d'un médicament à un sujet, comprenant :
l'application d'une pression mécanique sur le patch à succion muqueuse contenant le médicament avant ou pendant la mise en place d'une surface d'étanchéité muqueuse du patch à succion pour muqueuse sur une muqueuse du sujet ; et
la libération de la pression mécanique sur le patch de succion pour muqueuse pour créer une succion et une déformation de la muqueuse,
de sorte que le médicament migre à travers l'épithélium muqueux et pénètre dans la circulation systémique du sujet.

10. Patch de succion pour muqueuse pour son utilisation selon la revendication 9, dans lequel le patch de succion pour muqueuse adhère à la muqueuse avec une force d'environ 0,5 kPa à environ 200 kPa.

11. Patch de succion pour muqueuse pour son utilisation selon la revendication 9 ou 10, dans lequel le patch comprend :
au moins un bulbe formant au moins une cavité, chacune des au moins une cavité étant en communication fluidique avec au moins une ouverture, et une ou plusieurs des au moins une cavité renfermant le médicament ;
la surface d'étanchéité muqueuse délimitant la au moins une ouverture ; et
une surface d'application de pression opposée à la surface d'étanchéité muqueuse.
dans lequel le au moins un bulbe ayant une élasticité telle que, le au moins un bulbe étant dans un état expansé, lors de l'application de la pression mécanique sur la surface d'application de pression, le au moins un bulbe est déformé, et lorsque la pression mécanique est relâchée, le au moins un bulbe retrouve au moins partiellement son état expansé.

12. Le patch de succion pour muqueuse pour son utilisation selon l'une quelconque des revendications 9 à 11, dans lequel (a) le patch de succion pour muqueuse est appliqué sur la muqueuse pendant 1 minute à 10 heures, de préférence 5 minutes à 30 minutes ; et/ou (b) le médicament se trouve dans une formulation comprenant en outre au moins un excipient, de préférence le au moins un excipient comprend un agent favorisant la perméation.

13. Patch de succion pour muqueuse pour son utilisation selon l'une quelconque des revendications 9 à 12, dans lequel la muqueuse est une muqueuse buccale ou vaginale, de préférence la muqueuse est une muqueuse buccale.

14. Patch de succion pour muqueuse pour son utilisation selon l'une quelconque des revendications 9 à 13, dans lequel le médicament a un poids moléculaire d'environ 1 kDa à environ 50 kDa.

15. Le patch de succion pour muqueuse selon l'une quelconque des revendications 1 à 7, dans lequel la muqueuse est une muqueuse buccale ou vaginale, de préférence la muqueuse est une muqueuse buccale.
